# EUROPÄISCHE PATENTSCHRIFT

(11) **EP 2 931 775 B1**
(45) Veröffentlichungstag und Bekanntmachung des Hinweises auf die Patenterteilung: **21.04.2021**
(21) Anmeldenummer: 13808016.3
(22) Anmeldetag: 17.12.2013
(51) Int. Cl.: C08G 18/48, A61K 47/10, A61K 47/34, C08J 3/075, C08G 18/73, C08G 18/76, C08G 65/30, C08G 81/00, A61K 47/42, C08L 75/08, C08L 89/06

(54) **KETTENVERLÄNGERTE POLOXAMERE, DARAUS GEBILDETE THERMOREVERSIBLE HYDROGELE MIT BIOLOGISCHEN MATERIALIEN, UND MEDIZINISCHE ANWENDUNGEN DERSELBEN**
CHAIN-EXTENDING POLOXAMERS, THERMOREVERSIBLE HYDROGELS FORMED BY THEM WHICH INCLUDE BIOLOGICAL MATERIALS, AND MEDICINAL APPLICATIONS OF SAME
POLAXOMÈRES À CHAÎNE ALLONGÉE, HYDROGELS THERMORÉVERSIBLES FORMÉS À PARTIR DE CEUX-CI ET COMPORTANT DES MATIÈRES BIOLOGIQUES, ET LEURS APPLICATIONS MÉDICALES

(30) Priorität: 17.12.2012 DE 102012223416
(43) Veröffentlichungstag der Anmeldung: 21.10.2015
(73) Patentinhaber: Schieker, Matthias, 81929 München (DE)
(72) Erfinder: SCHIEKER, Matthias, 81929 München (DE); WIESE, Hinrich, 59423 Unna (DE); MOLL, Uta, 85622 Feldkirchen (DE)
(74) Vertreter: Bublak, Wolfgang
(86) Internationale Anmeldenummer: PCT/EP2013/076966
(87) Internationale Veröffentlichungsnummer: WO 2014/095915

(56) Entgegenhaltungen:
- WO-A2-97/05185
- WO-A2-2005/051428
- US-A- 3 767 040
- US-A- 4 039 724
- US-A- 4 423 201
- US-A- 4 608 201
- US-A1- 2005 175 573
- LAN P N ET AL: "Synthesis and characterization of segmented polyurethanes based on amphiphilic polyether diols", BIOMATERIALS, ELSEVIER SCIENCE PUBLISHERS BV., BARKING, GB, Bd. 17, Nr. 23, 1996, Seiten 2273-2280, XP004069063, ISSN: 0142-9612, DOI: 10.1016/0142-9612(96)00056-7
- JUN JIANG ET AL: "Rheology of Thermoreversible Hydrogels from Multiblock Associating Copolymers", MACROMOLECULES, Bd. 41, Nr. 10, Mai 2008 (2008-05), Seiten 3646-3652, XP055098469, ISSN: 0024-9297, DOI: 10.1021/ma800192m in der Anmeldung erwähnt
- VAN BOS M ET AL: "Hydrophilic polyurethanes for the preparation of drug delivery systems", ACTA PHARMACEUTICA TECHNOLOGICA, STUTTGART, DE, Bd. 33, Nr. 3, 1987, Seiten 120-125, XP002970383, ISSN: 0340-3157
- ELIAS VOLKMER ET AL: "Poloxamer-based hydrogels hardening at body core temperature as carriers for cell based therapies: in vitro and in vivo analysis", JOURNAL OF MATERIALS SCIENCE: MATERIALS IN MEDICINE, Bd. 24, Nr. 9, 28. Mai 2013 (2013-05-28), Seiten 2223-2234, XP055098779, ISSN: 0957-4530, DOI: 10.1007/s10856-013-4966-6

## Beschreibung

### Gebiet der Erfindung

Die Erfindung betrifft thermoreversible Hydrogele, die kettenverlängerte Poloxamere und biologisches Material umfassen. Darüberhinaus betrifft die Erfindung die medizinische Verwendung der thermoreversiblen Hydrogele, ein Applikationssystem umfassen ein thermoreversibles Hydrogel, sowie ein In-vitro-Verfahren zur Bildung einer Zusammensetzung auf einer Oberfläche.

### Hintergrund der Erfindung

Unter dem Begriff "Poloxamer" versteht man eine Klasse Polyoxyethylen-Polyoxypropylen-Blockcopolymeren (erstmals erwähnt in US 3,740,421), die auch bekannt sind unter dem Handelsnamen "Pluronic®" bzw. "Lutrol®" (Markenzeichen der BASF SE). Hierbei handelt es sich um Blockcopolymere aus hydrophilen Polyethylenglykol-Aussenblöcken und hydrophoberen Polypropylenglykol-Innenblöcken, d.h. Poly(ethylenoxid)-poly(propylenoxid)-poly(ethylenoxid)-triblockcopolymere, die allgemein durch die folgende Struktur zusammengefasst werden können:

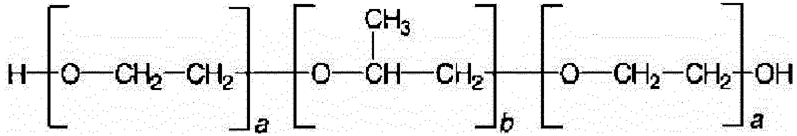

Diese Blockcopolymere bilden im Wasser durch Phasentrennung Sole bzw. Gele:
Die Polyethylenglykolblöcke lösen sich im Wasser, während die Polypropylenglykolblöcke sich aneinander lagern. Diesen Vorgang nennt man Mizellbildung. Während bei tieferer Temperatur (Solzustand) die Mizellen relativ ungeordnet vorliegen, bildet sich bei Erhöhung der Temperatur eine Ordnung dieser Mizellen aus, die zum Erstarren der Flüssigkeit führt (Gelzustand) (vgl. Alexandridis P., Holzwarth J.F., Hatton T.A. Micellization of Poly(ethylene oxide)-Poly(propylene oxide)-Poly(ethylene oxide) Triblock Copolymers in Aqueous Solutions: Thermodynamics of Copolymer Association. Macromolecules. 1994;27(9):2414-25). Daher werden solche Gele auch thermoreversible Hydrogele genannt.

Hydrogele auf Basis von Poloxameren sind seit längerem bekannt (vgl. Neville J., Swafford W.B.: Pluronics as a suppository base. Am. J. Pharm. Sci. Support. Public Health. 1960;132:301-303). Darüberhinaus wurde ein kettenverlängertes Polymer aus Poloxamer 407 (Pluronic® F 127) und Hexamethylendiisocyanat (HMDI) beschrieben (vgl. Jiang J., Malal R., Li C., Lin M.Y., Colby R.H., Gersappe D., Rafailovich M.H., Sokolov J.C., Cohn D.: Rheology of Thermoreversible Hydrogels from Multiblock Associating Copolymers. Macromolecules 2008;41 :3646-3652).

Da Poloxamere biologisch inert sind, wurden sie bald als Suppositorien für Pharmaka und mit dem Aufkommen der regenerativen Medizin auch als Zellträger eingesetzt (vgl. Kamil S.H., Eavey R.D., Vacanti M.P., Vacanti C.A., Hartnick C.: Tissueengineered cartilage as a graft source for laryngotracheal reconstruction - A pig model. Arch. Otolaryngol. 2004;130(9):1048-1051).

Der besondere Vorteil dieser Hydrogele liegt im beschriebenen thermosensitiven Verhalten. Dadurch ist eine minimalinvasive Applikation inkorporierter Zellen denkbar. Letztere können im flüssigen Zustand einfach eingebracht werden. Nach Implantation kommt es durch die Änderung der Temperatur zum Gelieren, wodurch die Zellen am gewünschten Ort gehalten werden (vgl. Cohn D., Lando G., Sosnik A., Garty S., Levi A.: PEO-PPO-PEO-based poly(ether ester urethane)s as degradable reverse thermo-responsive multiblock copolymers. Biomaterials 2006;27(9):1718-1727; Nguyen M.K., Lee D.S. Injectable biodegradable hydrogels. Macromol. Biosci.2010; 1 0(6):563-579).

Die Verwendung thermoreversibler Hydrogele ist z.B. bei der Behandlung von Verbrennungen vorteilhaft. Das Hydrogel ermöglicht die transdermale oder lokale Abgabe eines Wirkstoffs und erhält einen hohen Feuchtigkeitsgrad an der Hautoberfläche aufrecht. Dies verhindert eine Austrocknung. Darüberhinaus haftet das Hydrogel in beträchtlichem Maße am geschädigten Gewebe und weist eine gewisse Elastizität auf, wodurch eine Ablösung des Hydrogels vermieden und gleichzeitig aus der Wunde austretendes Sekret absorbiert wird. Allgemein fördern Hydrogele die Heilung, da sie an der Wundstelle schnell in den Gelzustand übergehen und Feuchtigkeit in der Wunde erhalten.

Während die meisten Poloxamere bei geringen Polymerkonzentrationen nur Gele mit schlechten mechanischen Eigenschaften ausbilden, kommt es bei kettenverlängerten Poloxameren zu einer höheren Stabilität der Gele. Erste Versuche zur Kettenverlängerung wurden mit Acrylsäureestern (Acrylaten) durchgeführt. Dadurch werden Einheiten mit C=C-Doppelbindungen an das Poloxamer geknüpft. Im Anschluss wurden chemische Additionsreaktionen durchgeführt, so dass mehrere Poloxamereinheiten verbunden werden konnten. Derivate der Acrylsäure sind physiologisch nicht unbedenklich. Wenn die Kettenverlängerung durch Diisocyanate durchgeführt wird, werden die einzelnen Poloxamere durch Urethaneinheiten verknüpft. Polyurethane werden aufgrund ihrer Gewebeverträglichkeit seit geraumer Zeit als medizinische Implantate genutzt. Nachteilig war bisher auch die mangelnde biologische Erkennbarkeit des Materials für die Zellen, die bisher nicht in der Lage waren, an dem Material zu adhärieren.

Der vorliegenden Erfindung liegt daher die Aufgabe zugrunde, kettenverlängerte Hydrogele ausgehend von Poloxameren bereitzustellen, die die im Stand der Technik bekannten Nachteile nicht aufweisen, und für medizinische Applikationen geeignet sind. Darüberhinaus liegt der Erfindung die Aufgabe zugrunde, thermoreversible Hydrogele bereitzustellen, die zur Freisetzung eines biologisch aktiven Mittels bzw. eines Wirkstoffs fähig sind. Daneben liegt der Erfindung die Aufgabe zugrunde, thermoreversible Hydrogele, die biologisches Material und lebende Zellen enthalten, wobei die Zellen an dem biologischen Material adhärieren, und therapeutische Anwendungen dafür zur Verfügung zu stellen.

### Zusammenfassung der Erfindung

Die obige Aufgabe wird gelöst durch die Bereitstellung des thermoreversiblen Hydrogels umfassend ein kettenverlängertes Poloxamer und biologisches Material gemäß den Ansprüchen 1 bis 15, die medizinische Verwendung des thermoreversiblen Hydrogels gemäß Ansprüchen 16 und 17, des Applikationssystems gemäß Anspruch 18 sowie dem *In-vitro-Verfahren* gemäß Anspruch 19.

### Kurze Beschreibung der Zeichnungen

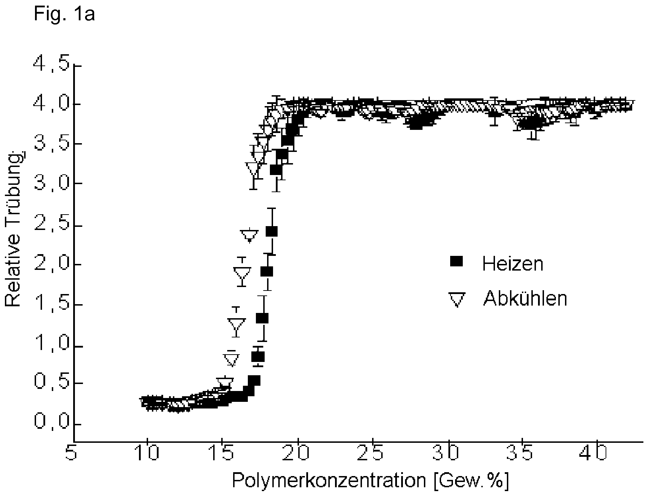

**Fig. 1a bis 1e** zeigen die physikalischen Eigenschaften von thermoreversiblen Hydrogelen, die aus mit Diisocyanaten kettenverlängerten Poloxameren hergestellt wurden, wobei mit Hexamethylendiisocyanat kettenverlängertes Poloxamer 403 bzw. Pluronic P 123 (PMT001), mit Butandiisocyanat kettenverlängertes Poloxamer 403 (PMT002; BDI-Hydrogel) und mit Methylenbiscyclohexyldiisocyanat kettenverlängertes Poloxamer 403 (PMT003) untersucht wurden. Diese Messungen wurden im Rahmen eines Kooperationsprojektes im Labor von Prof. Dr. W. Frieß am Zentrum für Pharmaforschung der Ludwig-Maximilians-Universität München durchgeführt.

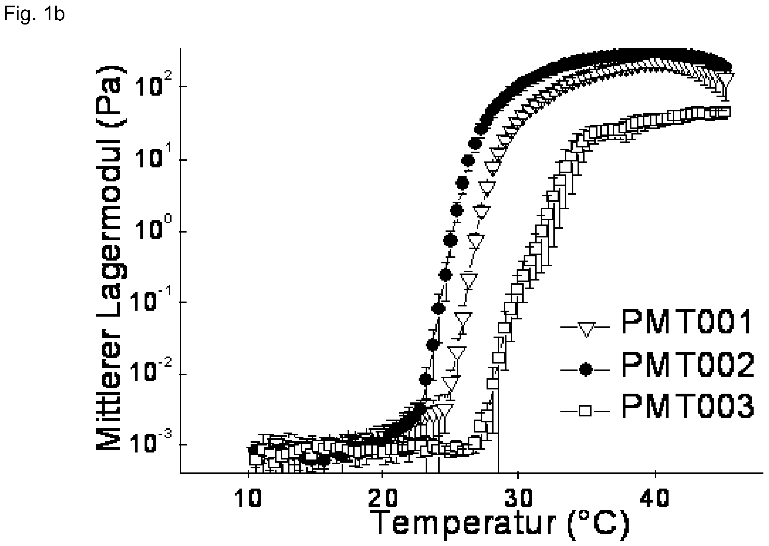

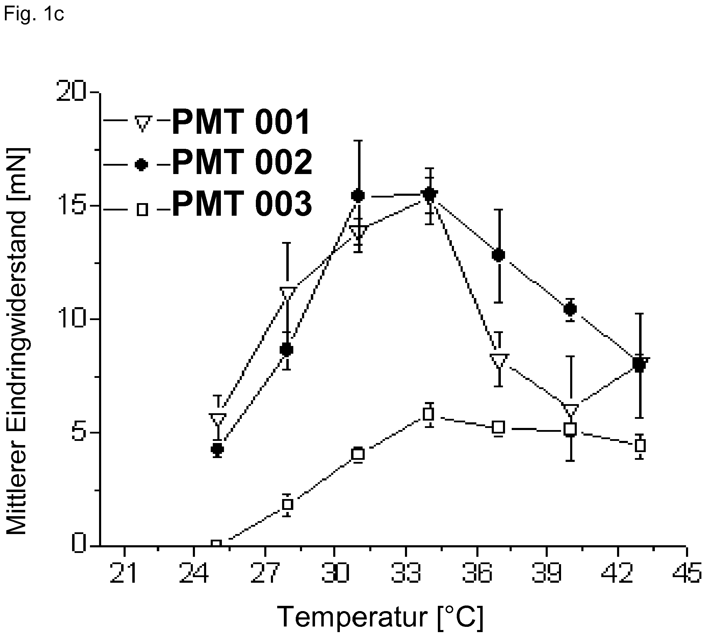

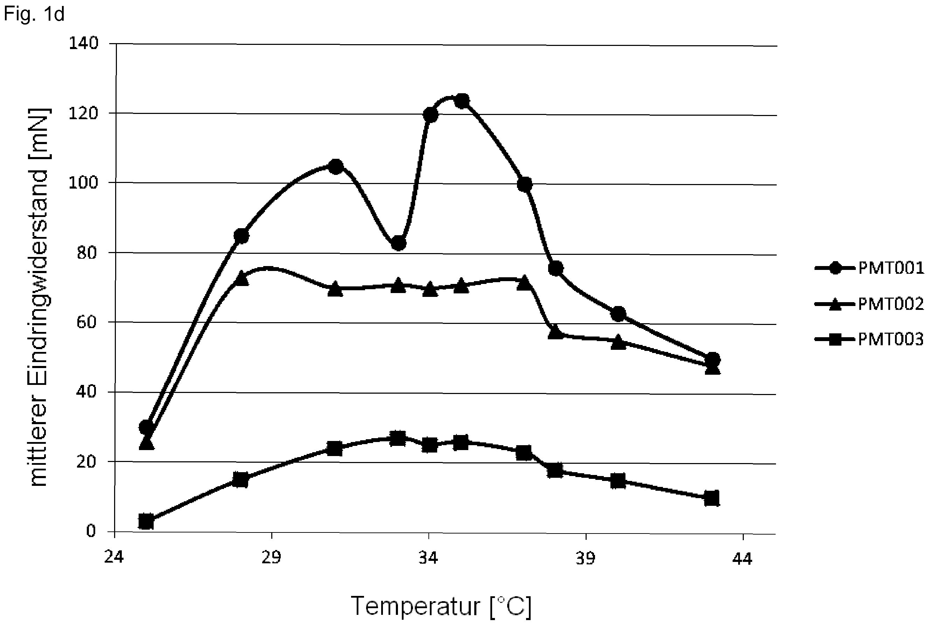

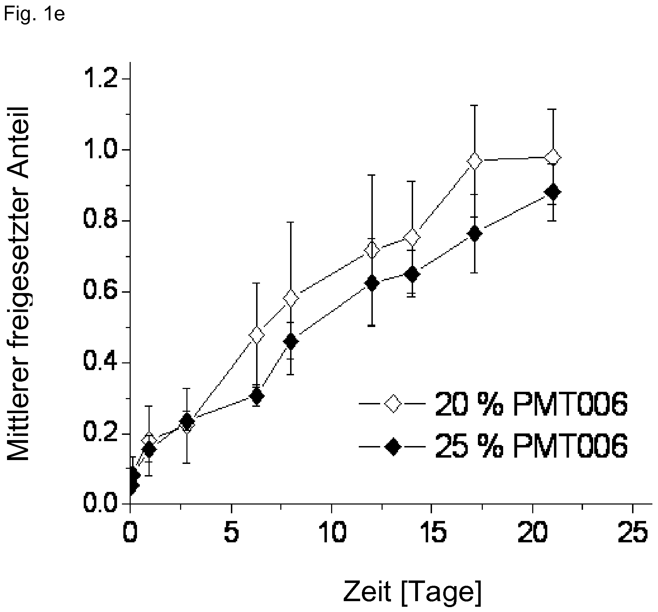

In **Fig. 1a** ist dabei die relative Trübung gegen die Polymerkonzentration in Gew.-% aufgetragen, in **Fig. 1b** der mittlere Lagermodul gegen die Temperatur, in **Fig.** 1c der mittlere Eindringwiderstand gegen die Temperatur, in **Fig. 1d** die Festigkeit der Gele gegen die Temperatur, und in **Fig. 1e** der mittlere freigesetzte Anteil für ein Protein als biologisches Material gegen die Zeit.

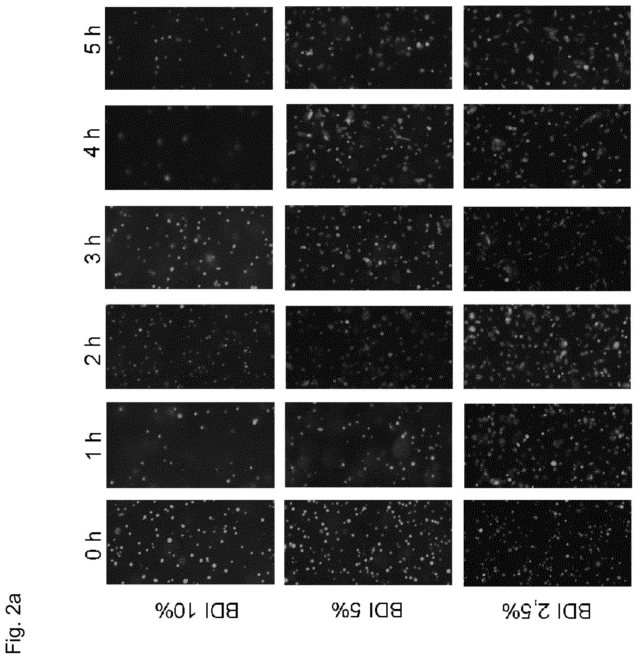

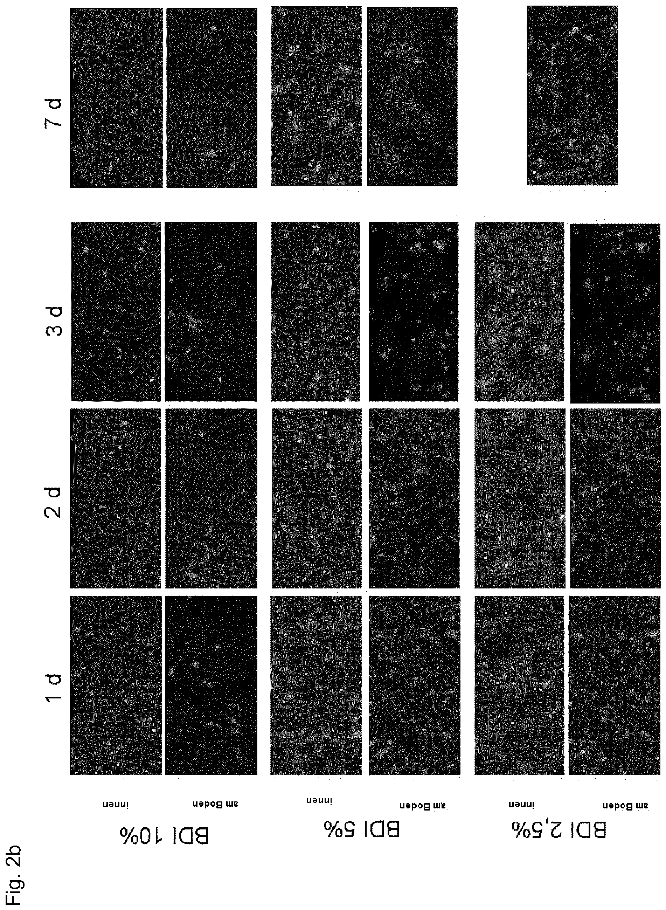

**Fig. 2a** **und** **2b** zeigen Aufnahmen, die das Zellüberleben bei unterschiedlichen Polymerkonzentrationen (2,5%, 5% und 10%) des thermoreversiblen Hydrogels aus mit Butandiisocyanat kettenverlängertem Poloxamer 403, im Folgenden auch als "BDI-Hydrogel" bezeichnet, veranschaulichen. Unterschiedliche Polymerkonzentrationen wurden dabei zunächst stündlich (in Fig. 1a die Reihen "0h" bis "5h"), dann täglich (in Fig. 2b die Reihen "1d", "2d"und "3d") und schliesslich noch nach einer Woche (in Fig. 2b die Reihe "7d") mittels "Live/Dead-Assay" untersucht.

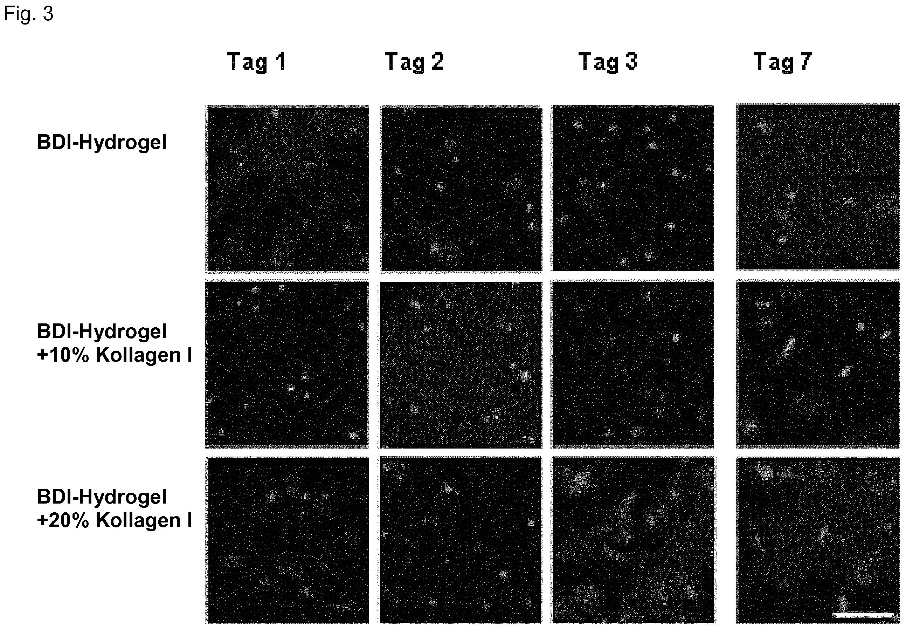

**Fig. 3** zeigt die Zellmorphologie in einem thermoreversiblen BDI-Hydrogel der Erfindung mit 10%, 20% bzw. ohne Kollagen, jeweils nach 1, 2, 3 und 7 Tagen. Der Maßstab beträgt 200 µm; alle Bilder wurden bei gleicher Vergrösserung aufgenommen.

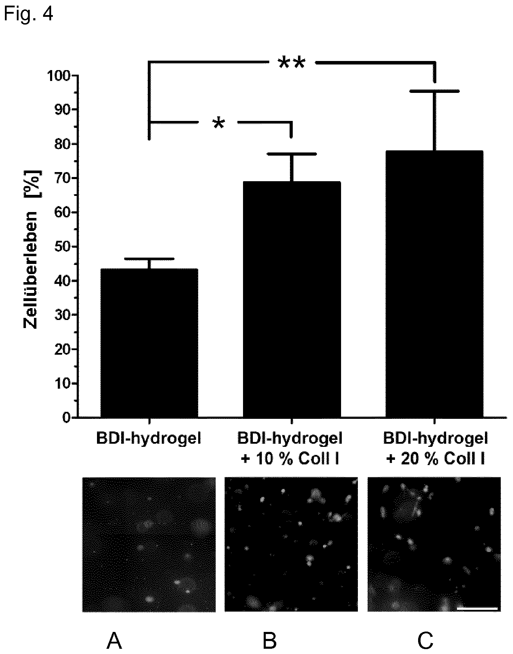

**Fig. 4** zeigt Mikroskopaufnahmen, die das Zellüberleben von eGFP-SCP1s nach 7 Tagen im thermoreversiblen BDI-Hydrogel der vorliegenden Erfindung mit bzw. ohne Kollagen I, veranschaulichen (MW ± SD, r=3, n=3). Kruskal-Wallis-Statistik mit multiplem Vergleichstest nach Dunn lieferte signifikante Unterschiede zwischen dem thermoreversiblen Hydrogel der Erfindung ohne (A) bzw. mit Kollagen I (B und C). Dabei ist a=0.05, *p < 0.05, sowie **p < 0.01. Beispielhafte Mikroskopbilder (am Boden, Vergrösserung 10x) nach Anfärben mit Propidiumiodid (PI); der Maßstab beträgt 200 µm.

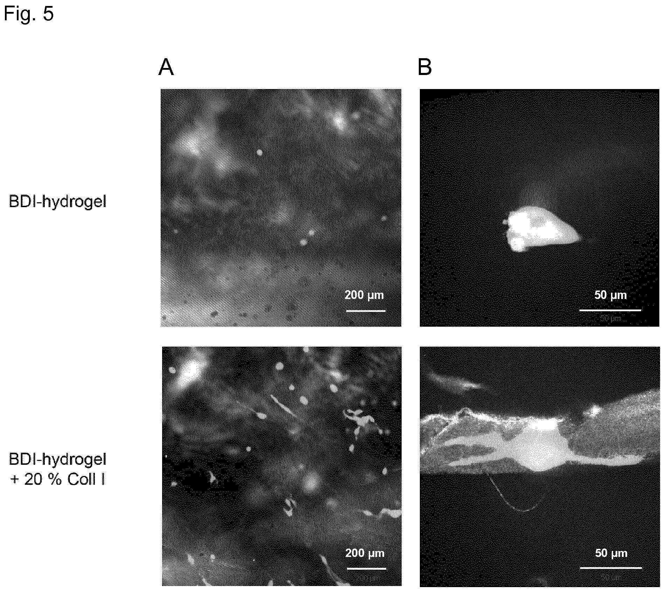

**Fig. 5** zeigt eGFP-SCP1s-Zellen nach 7 Tagen im thermoreversiblen BDI-Hydrogel der vorliegenden Erfindung (in der Abbildung als "BDI-hydrogel" bezeichnet) mit bzw. ohne 20 % Kollagen I, visualisiert mit konfokaler Laser-Scanning-Mikroskopie. Die Aufnahmen wurden mit einer Vergrösserung von 10x (Maßstab: 200 µm; Aufnahmen A) bzw. 63x (Maßstab: 50 µm; Aufnahmen B) vorgenommen.

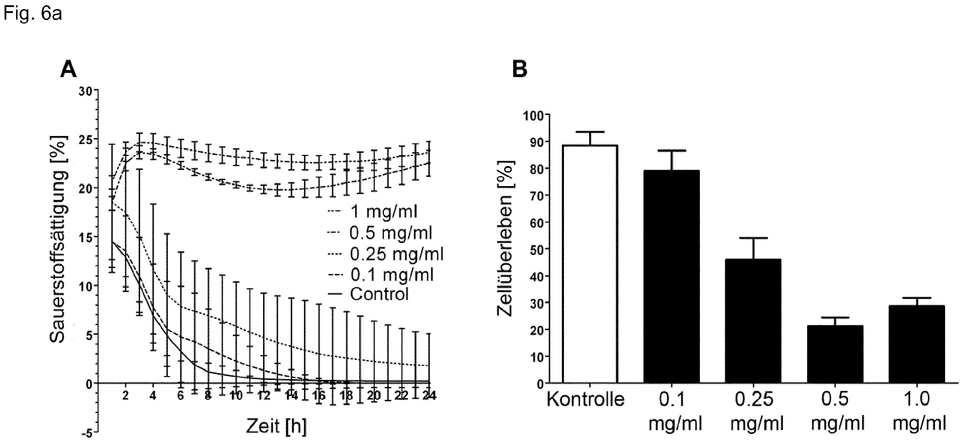

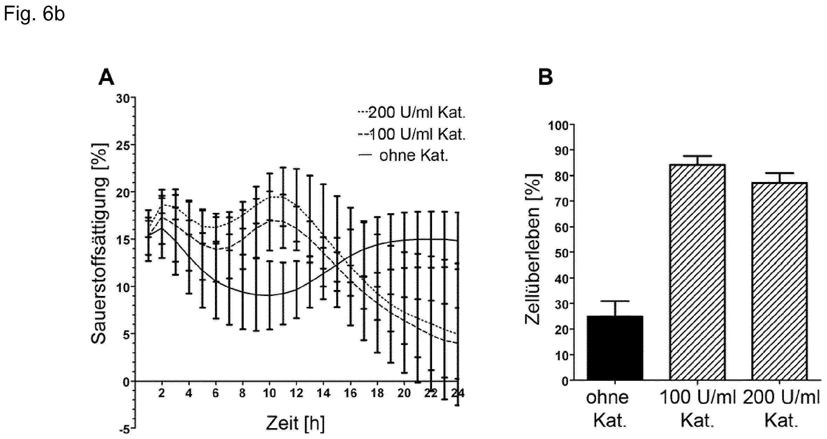

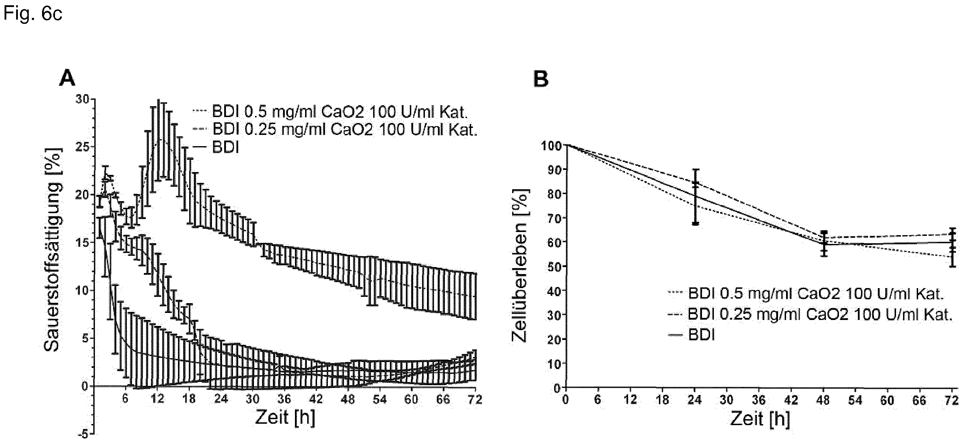

**Fig.** 6a zeigt die Sauerstoffsättigung (A) bzw. das Zellüberleben (B) nach 24 h Kultur in Spritzen in BDI-Hydrogel mit Ca02, "Kontrolle" steht für BDI-hydrogel ohne CaO₂ (MW±SD, r=3, n=1 ).
**Fig. 6b** zeigt die Sauerstoffsättigung (A) und Zellüberleben (B) nach 24 h Kultur in Spritzen in BDI-Hydrogel mit 0.25 mg/ml CaO₂ und verschiedenen Katalase-Konzentrationen (MW±SD, r=3, n=1 ).
**Fig.** 6c zeigt Sauerstoffsättigung (A) und Zellüberleben (B) nach 72 h Kultur in Spritzen in BDI-Hydrogel mit 0.5 mg/ml CaO₂ und 100 U/ml Katalase oder BDI-Hydrogel bei Konzentrationen von 0,25 mg/ml CaO₂ und 100 U/ml Katalase, (MW±SD, r=3, n=1 ).

### Genaue Beschreibung der Erfindung

Die vorliegende Erfindung stellt thermoreversible Hydrogele umfassend kettenverlängerte Poloxamere bereit, die aus Poloxameren unter Einsatz von Diisocyanaten hergestellt werden. Diese thermoreversiblen Hydrogele können in verschiedenster Weise auf medizinischem Gebiet verwendet werden.

Für die Erfindung geeignete Poloxamere, die zu thermoreversiblen Hydrogelen umgesetzt werden können, sind allgemein diejenigen, bei denen das Molekulargewicht des Poloxamers P mehr als 4000 Da beträgt. Erfindungsgemäß beträgt das Molekulargewicht des Polypropylenblocks mehr als 2350 Da. In einer bevorzugten Ausführungsform beträgt der Anteil an Poylethylenoxideinheiten im oben genannten Poloxamer bzw. den oben genannten Poloxameren 20% bis 80%, stärker bevorzugt 20% bis 70%, und besonders bevorzugt 20% bis 60%. Für die vorliegende Erfindung wird ein kettenverlängertes Poloxamer P*, hergestellt durch Umsetzung mindestens eines Poloxamers P mit einem Diisocyanat, bestehend aus zwei Polyoxyethylenblöcken PEG und einem Polyoxypropylenblock PPG, wobei das Molekulargewicht des Polypropylenoxid-Blocks von P grösser ist als 2350 Da, verwendet. Handelt es sich um mehr als ein Poloxamer, z.B. zwei verschiedene Poloxamere P und P', so erfüllt jedes Poloxamer die obigen Erfordernisse.

Zum Erhalt des erfindungsgemäßen kettenverlängerten Poloxamers wird in den vorgenannten Fällen bei einem Molekulargewicht des Poloxamers P von mindestens 11.000 Da und einem Anteil an Polyethylenoxid in P von mehr als 60% ein zusätzliches, vom vorgenannten Poloxamer verschiedenes Poloxamer verwendet. Zu den Poloxameren mit einem Molekulargewicht von 11.000 Da und einem Anteil an Polyethylenoxid von mehr als 60% zählt zum Beispiel Poloxamer 407 (Pluronic®F 127). In der vorliegenden Erfindung wird daher im Fall der Verwendung von Poloxamer 407 mindestens ein weiteres, von Poloxamer 407 unterschiedliches Poloxamer in Kombination verwendet.

In einer anderen, nicht erfindungsgemäßen Ausführungsform ist bei einem Molekulargewicht des oben genannten Poloxamers P von mindestens 11.000 Da und einem Anteil an Polyethylenoxid in **P** von mehr als 60% das Diisocyanat ausgewählt aus der Gruppe, bestehend aus Diisocyanaten der Formel O=C=N-(CH₂)ₙ-N=C=O mit n = 4, 5 oder n > 6, Isophorondiisocyanat, Methylendi(4,4'-isocyanato)cyclohexan (H₁₂-MDI), Lysindiisocyanat und aromatischen Diisocyanaten, bevorzugt Diphenylmethandiisocyanat (MDI). In einer bevorzugten Ausführungsform wird, falls Poloxamer 407 verwendet wird, dieses ggf. mit weiteren Poloxameren kombiniert und mit einem Diisocyanat, das aus den vor genannten ausgewählt wird, kettenverlängert.

Bevorzugt werden mindestens drei Wiederholungseinheiten mindestens eines Poloxamers P umgesetzt.

Die Poloxamereinheiten werden mit einem Diisocyanat zur Kettenverlängerung umgesetzt. Während aromatische Diisocyanate für die vorliegende Erfindung eingesetzt werden können, die allgemein im vorliegenden Gebiet aufgrund ihrer hohen Reaktivität bevorzugt werden, besteht ein Nachteil darin, dass sie zu cancerogenen Diaminen abgebaut werden können. Dagegen würde ein entsprechender Abbau bei aliphatischen Diisocyanaten beispielsweise zu Diaminen wie Putrescin oder Kadaverin führen, die in geringem Maße in den Stoffwechselprozessen des Körpers vorkommen.

Zudem sind die aliphatische Verbindungen auch beständig gegen andere Abbaureaktionen, weshalb sie in technischen Anwendungen eingesetzt werden, wenn Vergilbung o.ä. vermieden werden soll. Daher werden aliphatische und insbesondere lineare Diisocyanate erfindungsgemäß bevorzugt.

Die kommerziell verfügbaren Poloxamere (Pluronic® bzw. Lutrol®) unterscheiden sich im Verhältnis von Ethylenoxid- bzw. Polyethylenglykol- (PEG-)Einheiten a und Polypropylenoxid- bzw. (PPG-)Einheiten b, sowie in der Länge der Blöcke. Dies führt zu unterschiedlichem Verhalten in Wasser.

Falls ein Poloxamer wie oben beschrieben mit einem Diisocyanat umgesetzt wird, so enthält, insbesondere wenn es sich um ein einzelnes Poloxamer als Ausgangsmaterial handelt, das kettenverlängerte Poloxamer P* der vorliegenden Erfindung die folgende Struktureinheit: 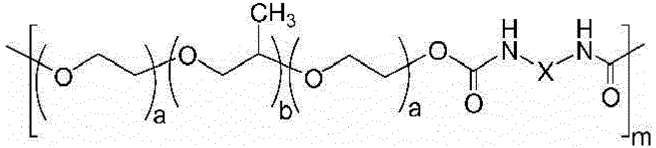 worin a und b jeweils ganze Zahlen zwischen 1 und 110 sind, und m ≥ 3 ist; a bezeichnet die Anzahl Wiederholungseinheiten des Poly(ethylenoxid)blocks PEG, und b bezeichnet die Anzahl Wiederholungseinheiten des Poly(propylenoxid)blocks PPG; hergestellt aus einem Diisocyanat der Formel O=C=N-X-N=C=O, wobei X der aliphatische oder aromatische Rest des Diisocyanats ist. Handelt es sich um zwei verschiedene Poloxamere (P und P') als Ausgangsmaterial, so besitzt das kettenverlängerte Poloxamer P* auch Struktureinheiten der Formel 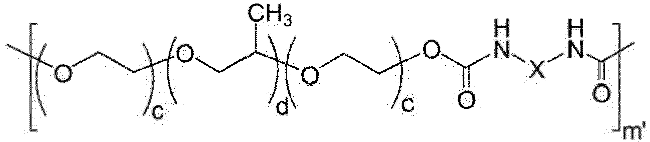 und/oder der Formel 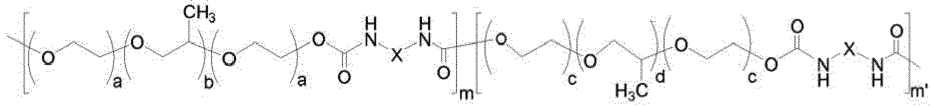 wobei a, b, m sowie X wie oben definiert sind, c die Anzahl Wiederholungseinheiten des Poly(ethylenoxid)blocks PEG, und d die Anzahl Wiederholungseinheiten des Poly(propylenoxid)blocks PPG jeweils im zweiten Poloxamer P' bezeichnet, wobei c und d jeweils ganze Zahlen zwischen 1 und 110 sind, und m' ≥ 3 ist.

In einer bevorzugten Ausführungsform ist das bzw. sind die kettenverlängerte(n) Poloxamer(e) P ausgewählt aus einem bzw. mehreren der Poloxamere 272, 278, 331, 333, 334, 335, 338, 401, 402, 403 und 407 (Handelsbezeichnungen: Pluronic® bzw. Lutrol® L 92, F 98, L 101, P 103, P 104, P 105, F 108, L 121, L 122, P 123 sowie F127). Das Poloxamer 407 wird in Kombination mit einem oder mehreren anderen Poloxamer(en) eingesetzt.

In einer weiter bevorzugten Ausführungsform wird Poloxamer 407 in Kombination mit weiteren Poloxameren mit linearen Diisocyanaten der Formel O=C=N-(CH₂)ₙ-N=C=O mit n = 4, 5 oder n > 6 umgesetzt, oder mit aliphatischen Diisocyanaten wie Isophorondiisocyanat, Methylendi(4,4'-isocyanato)cyclohexan (H₁₂-MDI) bzw. Lysindiisocyanat, oder aromatischen Diisocyanaten, bevorzugt Diphenylmethandiisocyanat (MDI).

Die Poloxamere der vorliegenden Erfindung können allgemein flüssig, pastenartig oder fest sein. Beispielsweise sind unter gewöhnlichen Bedingungen die Poloxamere 272, 331, 401 und 402 (bzw. die mit "L" bezeichneten Pluronic-Typen) flüssig, die Poloxamere 333, 334, 335 und 403 (bzw. die mit "P" bezeichneten Pluronic-Typen) pastenförmig, und die Poloxamere 278, 668 und 407 (bzw. die mit "F" bezeichneten Pluronic-Typen) fest.

Besonders bevorzugt sind gemäß der Erfindung thermoreversible Hydrogele auf Basis von Poloxamer 403, 407 und 338.

Bislang ist hauptsächlich Poloxamer 407 bzw. "Pluronic® F 127" (entsprechend der obigen allgemeinen Formel mit a=100, b=65; d.h. [PEG]₆₅ [PPG]₁₀₀[PEG]₆₅ in Hydrogelen für medizinische und pharmazeutische Zwecken eingesetzt worden.

Erfindungsgemäß können auch mehrere Poloxamere P zu einem kettenverlängerten Poloxamer P* miteinander kombiniert werden. Bevorzugt sind hierbei kettenverlängerte Poloxamere P* aus zwei oder drei verschiedenen Poloxameren.

Erfindungsgemäß werden die kettenverlängerten Poloxamere P* in thermoreversiblen Hydrogelen eingesetzt. Das thermoreversible Hydrogel der vorliegenden Erfindung ist ein thermoreversibles Hydrogel, das
(a) das oben genannte kettenverlängerte Poloxamer P* sowie
(b) ein biologisches Material, ausgewählt aus der Gruppe, bestehend aus einem antibiotischen, antimikrobiellen oder antimykotischen Wirkstoff, Proteinen, Glukosaminoglykanen, Lysozym und einer Polyaminosäure umfasst.

Das thermoreversible Hydrogel der vorliegenden Erfindung ist erhältlich durch
(a) Bildung des oben genannten kettenverlängerten Poloxamers P*, und
(b) Mischen des kettenverlängerten Poloxamers mit einem biologischen Material, ausgewählt aus der Gruppe, bestehend aus einem antibiotischen, antimikrobiellen oder antimykotischen Wirkstoff, Proteinen, Glukosaminoglykanen, Lysozym und einer Polyaminosäure.

Als Diisocyanate werden aromatische und aliphatische, insbesondere lineare Diisocyanate, die sich als Kettenverlängerungsmittel als besonders vorteilhaft erwiesen haben, bevorzugt.

Wie eingangs erwähnt, ist gemäß dem Stand der Technik die mangelnde biologische Erkennbarkeit des Materials für die Zellen nachteilig, da letztere bisher nicht in der Lage waren, an dem Material zu adhärieren. Die Erfindung basiert insbesondere auf der beispielhaften Idee, das auf einem kettenverlängertem Poloxamer basierende Hydrogel mit Kollagen als einem biologischen Material zu versetzen, und der Entdeckung der überraschend vorteilhaften Eigenschaften des daraus hergestellten thermoreversiblen Hydrogels. Gemäß einer bevorzugten Ausführungsform kann dazu z.B. fein gemahlenes oder lösliches Kollagen als biologisches Material verwendet werden. In Laborversuchen zeigte sich, dass die Zellen in einem gewissen Toleranzbereich eine ausgebreitete Zellmorphologie aufweisen, was auf eine Adhärenz an Kollagen schliessen lässt.

Erfindungsgemäß wurde gefunden, dass z.B. auch aus dem relativ hydrophoben Poloxamer 403 (Pluronic® P 123) in geringen Konzentrationen vielseitig einsetzbare thermoreversible Hydrogele mit entsprechenden physikalischen Eigenschaften hergestellt werden können, besonders wenn diese, wie oben beschrieben, mit linearen bzw. aliphatischen Diisocyanaten kettenverlängert werden. Diese Hydrogele haben sich als Applikationssystem für Zellen und hochmolekulare Proteine besonders bewährt. Die Hydrogele gemäß der vorliegenden Erfindung zeigen stark verbesserte Eigenschaften gegenüber Hydrogelen, die auf bekannten kettenverlängerten Poloxameren basieren. So liegt ein besonderer Vorteil der thermoreversiblen Hydrogele auf Basis von Poloxamer 403 gegenüber denen auf Basis von ausschließlich Poloxamer 407 (Pluronic® F 127) darin, dass sie *in vivo* länger beständig sind.

Um ein längeres Zellüberleben und eine biologische Funktionalität der integrierten Zellen zu erhalten, werden erfindungsgemäß die Hydrogele mit biologischem Material modifiziert. Dadurch erhalten die Zellen Adhäsionspunkte, an denen sie mittels Integrinbindung adhärieren können. Eine solche Adhäsion ist für die Aktivität der Zellen Voraussetzung (vgl. Popov C., Radic T., Haasters F., Prall W.C., Aszodi A., Gullberg D. et al.: Integrins alpha2beta1 and alpha11 beta1 regulate the survival of mesenchymal stem cells on collagen I. Cell Death Dis. 2011; 2: e186).

Erfindungsgemäß geeignete biologische Materialien sind ausgewählt aus der Gruppe, bestehend aus einem antibiotischen, antimikrobiellen oder antimykotischen Wirkstoff, Proteinen, Glukosaminoglykanen, Lysozym und einer Polyaminosäure. Der antibiotische, antimikrobielle oder antimykotische Wirkstoff ist bevorzugt ausgewählt aus β-Lactam-Antibiotika, Tetracyclinen, Aminoglykosiden, Makroliden, Virustatika, Allylaminen, antimykotisch wirksamen Antibiotika, Imidazolen und Derivaten davon. Die Polyaminosäure ist bevorzugt ausgewählt aus natürlichen und synthetischen Polyaminosäuren.

In einer bevorzugten Ausführungsform ist das biologische Material ausgewählt aus der Klasse der Proteine.

Erfindungsgemäß wird das Protein bevorzugt ausgewählt aus der Gruppe, bestehend aus einem das Zellwachstum und die Zelldifferenzierung hemmenden oder stimulierenden Protein; oder aus der Gruppe, bestehend aus Fibrin, Gelatine, Kollagenen, und knochenmorphogenen Proteinen (BMP). Das Protein kann auch ein Wachstumsfaktor sein, und ist bevorzugt ausgewählt aus der Gruppe, bestehend aus Insulin-ähnlichem Wachstumsfaktor (IGF), Transforming growth factor (TGF), Platelet Derived Growth Factor (PDGF), Epidermalem Wachstumsfaktor (EGF), FibroblastenWachstumsfaktor (FGF), Granulocyte-Macrophage Colony Stimulating Factor (GMCSF), Vascular Endothelial Growth Factor (VEGF), Hepatocyte Growth Factor (HGF), Interleukin-1 B (IL-1 B), Interleukin-8 (IL-8), Nervenwachstumsfaktor (NGF), und hämatopoetischen Wachstumsfaktoren wie Erythropoietin und koloniestimulierenden Faktoren wie G-CSF.

In einer bevorzugten Ausführungsform ist das biologische Material ausgewählt aus ß-Lactam-Antibiotika, Tetracyclinen, Aminoglykosiden, Makroliden, Virustatika, Allylaminen, antimykotisch wirksamen Antibiotika, Imidazolen, sowie Derivaten davon, Epi- dermalemWachstumsfaktor (EGF), TGF-ß-(transforming growth factor beta)-Superfamilie, bevorzugt BMP (bone morhogenetic protein)- und GOF (growth differentiation factor)-Familien, stärker bevorzugt BMP2, BMP7 und GDF-5.

In einer bevorzugten Ausführungsform besteht das biologische Material aus löslichem Kollagen, fein gemahlenem Kollagen, Gelatine, menschlichem Blut, tierischem Blut, Komponenten menschlichen oder tierischen Blutes.

In einer bevorzugten Ausführungsform besteht das biologische Material aus Kollagen Typ I, II oder III.

In einer bevorzugten Ausführungsform ist das biologische Material bzw. Protein ein Hormon oder Enzym, stärker bevorzugt Follistatin oder Myostatin
Das Protein kann auch ein Immunoglobulin oder ein Antikörper sein, bevorzugt ausgewählt aus der Gruppe, bestehend aus IgA, IgD, IgE, IgM, IgG, IgY, und IgW.

Darüberhinaus kann, falls das biologische Material ein Glukosaminoglykan ist, Hyaluronan (Hyaluronsäure) verwendet werden.

Das thermoreversible Hydrogel der vorliegenden Erfindung kann auch lebende Zellen umfassen.

Die vorgenannten Zellen sind bevorzugt ausgewählt aus der Gruppe, bestehend aus mononukleären Zellen, insbesondere mesenchymalen Stammzellen, Vorläuferzellen und daraus differenzierten Zellen wie Osteoblasten, Adipozyten, Chondrozyten, Fibroblasten, Epithelzellen, Myoblasten, Tendozyten, oder mononukleären hämatopoetischen Stamm- und Vorläuferzellen und daraus differenzierten Zellen wie z.B. Immunzellen, oder multinukleären Zellen wie Riesenzellen, Makrophagen und Osteoklasten.

In einer weiteren bevorzugten Ausführungsform sind die Zellen genetisch modifiziert oder werden im Gel modifiziert.

In einer bevorzugten Ausführungsform kann das thermoreversible Hydrogel auch einen Tracer enthalten. Hierbei sind Tracer aus radioaktiven Substanzen, die mittels **PET** oder SPECT sichtbar gemacht werden können, bevorzugt. Beispiele für solche Tracer sind Radioisotope wie Tritium, ¹⁴C, ¹⁸F, ³²P, ³⁵S, ¹¹¹ln und ¹²³I.

Überraschend wurde nun gefunden, dass bei den thermoreversiblen Hydrogelen der Erfindung aufgrund der Verbindung mit biologischem Material eine ausgebreitete Zellmorphologie inkorporierter Zellen entsteht, die auf die Ausbildung von Integrinbindungen am biologischen Material (z.B. Kollagen) schliessen lässt, und gleichzeitig die mechanischen vorteilhaften Eigenschaften des thermoreversiblen Hydrogels kaum verändert werden.

Das thermoreversible Hydrogel der vorliegenden Erfindung ermöglicht einen vielseitigen Einsatz auf medizinischem Gebiet. Das erfindungsgemäße thermoreversible Hydrogel kann medizinisch verwendet werden, wobei die medizinische Verwendung Arzneimittelabgabe oder eine biomedizinische Anwendung ist.

In einer erfindungsgemäßen Ausführungsform stellt die Erfindung ein Applikationssystem bereit, welches das oben genannte thermoreversible Hydrogel umfasst. In dieser Ausführungsform umfasst das vorgenannte Applikationssystem bevorzugt auch ein therapeutisches Mittel, das Zell-/Gewebewachstum stimuliert oder hemmt oder Zellen abtötet.

Eine Vielzahl therapeutischer Mittel kann unter Verwendung der thermoreversiblen Hydrogele der vorliegenden Erfindung zugeführt werden. Beispiele umfassen die für das biologische Material oben genannten Mittel, sowie allgemein synthetische anorganische und organische Verbindungen, Proteine und Peptide, Polysaccharide und andere Zucker, Lipide, Ganglioside und Nucleinsäuresequenzen mit therapeutischen, prophylaktischen oder diagnostischen Aktivitäten. Nucleinsäuresequenzen umfassen Gene, Antisense-Moleküle, die an komplementäre DNA unter Hemmung der Transkription binden, und Ribozyme. Die einzuarbeitenden therapeutischen Mittel können eine Vielzahl biologischer Aktivitäten aufweisen, beispielsweise vasoaktive Mittel, neuroaktive Mittel, Hormone, Antikoagulantien, Immunmodulatoren, cytotoxische Mittel, Antibiotika, antivirale Mittel, Antisense-Mittel, Antigene und Antikörper. Proteine, die Antikörper oder Antigene umfassen, Anticaline oder ähnliche künstliche Proteine, die zur Bindung von Antigenen befähigt sind, können ebenfalls zugeführt werden. Proteine sind derart definiert, dass sie aus 100 Aminosäureresten oder mehr bestehen; Peptide sind weniger als 100 Aminosäurereste. Falls nicht anders angegeben, bezeichnet der Ausdruck Protein sowohl Protein als auch Peptide. Beispiele umfassen Insulin, Parathormon, Parathormonfragmente, Irisin, Myostatin, Follistatin und andere Hormone.

Spezielle therapeutische Mittel umfassen Antibiotika, antivirale Mittel, sowohl steroidale als auch nicht-steroidale entzündungshemmende Mittel, Antineoplastika, Antispasmodika einschließlich Kanalblockern, Modulatoren von Zelle/extrazelluläre Matrix-Interaktionen, die Zellwachstumsinhibitoren und Antiadhäsionsmoleküle um- fassen, Enzyme und Enzyminhibitoren, Antikoagulantien und/oder Antithrombosemittel, Wachstumsfaktoren, DNA, RNA, Inhibitoren der DNA-, RNA-oder Proteinsynthese, die Migration, Proliferation und/oder das Wachstum von Zellen modulierende Verbindungen, Vasodilatationsmittel und andere Arzneimittel, die üblicherweise zur Behandlung von Gewebeläsionen verwendet werden. Spezielle Beispiele für diese Verbindungen umfassen Angiotensin Converting Enzyme-Inhibitoren, Prostacyclin, Heparin, Salicylate, Nitrate, Calciumkanalblocker, Streptokinase, Urokinase, Gewebeplasminogenaktivator (TPA) und anisoylierten Plasminogenaktivator (TPA) und anisoylierten Plasminogen-Streptokinase-Aktivatorkomplex (APSAC), Colchicin und Alkylierungsmittel und Aptomere. Spezielle Beispiele für Modulatoren von Zellinteraktionen umfassen Interleukine, Plättchenwachstumsfaktor, sauren und basischen Fibroblastenwachstumsfaktor (FGF), Transformationswachstumsfaktor ß; (TFG ß), epidermalen Wachstumsfaktor (EGF), insulinähnlichen Wachstumsfaktor und Antikörper hierfür. Spezielle Beispiele für Nucleinsäuren umfassen Gene und cDNAs mit Codierung für Proteine, Expressionsvektoren, Antisense- und andere Oligonucleotide, wie Ribozyme, die zur Regulierung oder Verhinderung einer Genexpression verwendet werden können. Spezielle Beispiele für andere biologisch aktive Mittel umfassen modifizierte Komponenten der extrazellulären Matrix oder deren Rezeptoren und Lipid- und Cholesterinsequestrierungsmittel.

Beispiele für Proteine als therapeutische Mittel umfassen ferner Cyctokine, wie Interferone und Interleukine, Proteine und koloniestimulierende Faktoren. Kohlehydrate als therapeutische Mittel umfassen Sialyl Lewis^{x}, für das gezeigt wurde, dass es an Rezeptoren für Selectine unter Hemmung einer Entzündung bindet. Ein "zuführbares Wachstumsfaktoräquivalent" (abgekürzt DGFE), ein Wachstumsfaktor für eine Zelle oder ein Gewebe, kann verwendet werden, der breit derart konstruiert ist, dass er Wachstumsfaktoren, Cytokine, Interferone, Interleukine, Proteine, koloniestimulierende Faktoren, Gibberelline, Auxine und Vitamine umfasst; ferner Peptidfragmente oder andere aktive Fragmente der obigen umfasst; und ferner Vektoren, d.h. Nucleinsäurekonstrukte mit der Fähigkeit zur Synthese derartiger Faktoren in den Zielzellen, ob durch Transformation oder transiente Expression, umfasst; und ferner Effektoren umfasst, die die Synthese derartiger Faktoren im Gewebe stimulieren oder unterdrücken, wobei diese natürliche Signalmoleküle, Antisense- und Dreifachhelixnucleinsäuren und dgl. umfassen. Beispiele für DGFEs sind vaskulärer endothelialer Wachstumsfaktor (VEGF), Endothelzellenwachstumsfaktor (ECGF), basischer Fibroblastenwachstumsfaktor (bFGF), Knochenwachstumsprotein (BMP) und Plättchenwachstumsfaktor (PDGF) und DNAs mit Codierung derselben. Beispiele für Gerinnselauflösungsmittel sind Gewebeplasminogenaktivator, Streptokinase, Urokinase und Heparin.

Als therapeutische Mittel können auch Arzneimittel mit antioxidierender Aktivität (d.h., die aktiven Sauerstoff zerstören oder dessen Bildung verhindern) in dem thermoreversiblen Hydrogel der vorliegenden Erfindung bereitgestellt werden, die beispielsweise bei der Verhinderung von Adhäsionen verwendbar sind. Beispiele umfassen Superoxiddismutase oder andere Proteinarzneimittel umfassen Katalasen, Peroxidasen und allgemeine Oxidasen oder oxidative Enzyme, wie Cytochrom P450, Glutathionperoxidase und andere native oder denaturierte Hämoproteine.

Als therapeutische Mittel können auch Säugerstressreaktionsproteine oder Hitzeschockproteine, wie Hitzeschockprotein 70 (hsp 70) und hsp 90 oder die Stimuli, die die Expression von Stressreaktionsproteinen oder Hitzeschockprotein hemmen oder verringern, beispielsweise Flavonoide, in dem Hydrogel bereitgestellt werden.

In einer weiteren bevorzugten Ausführungsform umfasst das vorgenannte Applikationssystem aus dem thermoreversiblen Hydrogel der vorliegenden Erfindung des Weiteren eine sauerstofffreisetzende Substanz. Die sauerstofffreisetzende Substanz liegt dabei bevorzugt in Kombination mit Enzymen vor. Beispiele für das Enzym umfassen Superoxiddismutase, Katalasen, Peroxidasen und allgemeine Oxidasen oder oxidative Enzyme wie Cytochrom P450, Glutathionperoxidase und andere native oder denaturierte Hämoproteine.

Ein Applikationssystem der vorliegenden Erfindung mit sauerstofffreisetzenden Stoffen wird in Beispiel 8 unten erläutert.

Ziel des Zusatzes von sauerstofffreisetzenden Stoffen zum Gel ist es, das Absterben der Zellen aus Sauerstoffmangel in größeren Konstrukten zu verhindern, bis sich neue Blutgefässe gebildet haben, welche die Zellen versorgen können. Die Versorgung über Diffusion funktioniert maximal über wenige Millimeter. Wenn ausschließlich Peroxide und Zellen ins thermoreversible Hydrogel der Erfindung gegeben werden, sterben die Zellen durch die Wirkung von gebildetem Wasserstoffperoxid. Wird jedoch ein peroxidabbauendes Enzym wie z. B. Katalase zugegeben, kann der Zelltod verhindert werden.

Die vorliegende Erfindung stellt darüberhinaus das oben genannte thermoreversible Hydrogel zur Verwendung bei der Herstellung eines Medikaments zur Behandlung eines medizinischen Zustands bereit, wobei das Medikament zur Applikation einer wässrigen Lösung des thermoreversiblen Hydrogels auf Gewebe *in vivo* dient.

In einer bevorzugten Ausführungsform umfasst die vorgenannte wässrige Lösung eine Lösung oder Suspension eines biologisch aktiven Materials.

In einer bevorzugten Ausführungsform ist der vorgenannte medizinische Zustand eine Verbrennung oder Abtragung der Haut.

In einer anderen bevorzugten Ausführungsform ist der vorgenannte medizinische Zustand ein durch einen chirurgischen Eingriff gestörtes Gewebe. In einer bevorzugten Ausführungsform ist der vorgenannte chirurgische Eingriff eine Vertebro- oder Kyphoplastie.

Die Applikation des thermoreversiblen Hydrogels der vorliegenden Erfindung kann auch mit bekannten Techniken wie Laparoskopie und Endoskopie erfolgen. Es können auch bekannte Kathetersysteme, wie sie z. B. in den US-Patenten 5,328,471 oder 5,213,580 beschrieben sind, angewendet werden.

In einer bevorzugten Ausführungsform wird die vorgenannte medizinische Verwendung dadurch charakterisiert, dass der chirurgische Eingriff durch die Kanüle eines Trokars durchgeführt wird
In einer bevorzugten Ausführungsform wird bei der vorgenannten medizinischen Verwendung das thermoreversible Hydrogel in einem pharmazeutisch akzeptablen Träger auf Gewebe appliziert. Beispielhafte Träger sind Kochsalzlösung oder phosphatgepufferte Kochsalzlösung.

In einer bevorzugten Ausführungsform wird bei der vorgenannten medizinischen Verwendung das thermoreversible Hydrogel in einem pharmazeutisch akzeptablen Träger zur parenteralen Verabreichung bereitgestellt.

In einer bevorzugten Ausführungsform befindet sich bei der vorgenannten medizinischen Verwendung das thermoreversible Hydrogel auf einer Oberfläche eines biologischen Gewebes, oder auf einer Oberfläche einer medizinischen Vorrichtung. Darüberhinaus kann sich das thermoreversible Hydrogel zwischen gegenüberliegenden Oberflächen befinden, wodurch eine Tendenz zum Hatten der Oberflächen aneinander erzeugt wird.

In einer bevorzugten Ausführungsform ist bei der vorgenannten medizinischen Verwendung das biologische Gewebe ein Organ. In der vorgenannten Ausführungsform wird bei der vorgenannten medizinischen Verwendung das Organ bevorzugt ausgewählt aus Haut, einem viszeralen Organ, Gewebe des muskuloskelettalen Apparates, bevorzugt Knochen, Knorpel, Bindegewebe, bevorzugt Sehnen, und Fettgewebe.

Schliesslich stellt die vorliegende Erfindung ein In-vitro-Verfahren zur Bildung einer Zusammensetzung auf einer Oberfläche bereit, das die Applikation einer wässrigen Lösung des oben genannten thermoreversiblen Hydrogels, von Gemischen derselben, oder des oben genannten medizinischen Applikationssystems auf die Oberfläche umfasst.

Bevorzugt umfasst das In-vitro-Verfahren die Applikation einer wässrigen Lösung des oben genannten, lebende Zellen enthaltenden thermoreversiblen Hydrogels.

Weiter bevorzugt wird das In-vitro-Verfahren unter Verwendung der vorgenannten Applikationssysteme, insbesondere einem Applikationssystem, das lebende Zellen und eine sauerstofffreisetzende Substanz und ein Enzym umfasst, durchgeführt. Bevorzugt wird als sauerstofffreisetzende Substanz dabei Calciumperoxid verwendet. Als Enzym wird z.B. Katalase bevorzugt.

In einer besonders bevorzugten Ausführungsform des vorgenannten In-vitro-Verfahrens ist das thermoreversible Hydrogel der Erfindung hergestellt aus Poloxamer 403 (Pluronic® P 123).

Die folgenden Beispiele veranschaulichen die Herstellung und Eigenschaften der kettenverlängerten Poloxamere bzw. der daraus erhaltenen thermoreversiblen Hydrogele gemäß der vorliegenden Erfindung.

### Beispiele

### Beispiel 1: Herstellung von mit Hexamethylendiisocyanat kettenverlängertem Poloxamer

In einen Glaskolben mit Rückflusskühler werden 17,4 g Poloxamer 403 (Pluronic® P 123; 3 mmol) auf 80 g mit trockenem Toluol aufgefüllt. Man rührt mit Magnetrührfisch bei ca. 45°C, bis eine klare Lösung vorliegt. 20 mL Toluol werden abdestilliert, um Wasserspuren zu entfernen. Anschließend gibt man jeweils mit einer Spritze 0,49 g Hexamethylendiisocyanat (HDI, 2,92 mmol) und eine Lösung von 120 µL 1,8-Diazabicyclo[5.4.0]undec-7-en (DBU) in 2 mL Toluol zu. Die Temperatur wird auf 55°C erhöht. Nach 2 Std. entfernt man das Toluol am Rotationsverdampfer. Anschließend trocknet man bei 45°C (Wasserbad) am Feinvakuum bis zur Gewichtskonstanz.

### Beispiel 2: Herstellung von mit Butandiisocyanaktettenverlängertem Poloxamer

In einen Glaskolben mit Rückflusskühler werden 17,4 g Poloxamer 403 (3 mmol) auf 80 g mit trockenem Toluol aufgefüllt. Man rührt mit Magnetrührfisch bei ca. 45°C, bis eine klare Lösung vorliegt. 20 mL Toluol werden abdestilliert, um Wasserspuren zu entfernen. Anschließend gibt man jeweils mit einer Spritze 0,373 g Butandiisocyanat (BDI, 2,92 mmol) und eine Lösung von 120 µL 1,8-Diazabicyclo[5.4.0]undec-7-en (DBU) in 2 mL Toluol zu. Die Temperatur wird auf 55°C erhöht. Nach 2 Std. entfernt man das Toluol am Rotationsverdampfer. Anschließend trocknet man bei 45°C (Wasserbad) am Feinvakuum bis zur Gewichtskonstanz.

### Beispiel 3: Herstellung von mit Methylendiphenyldiisocyanat kettenverlängertem Poloxamer

In einen Glaskolben mit Rückflusskühler werden 17,4 g Poloxamer 403 (3 mmol) ein- gewogen. Es werden 63 g trockenes Toluol zugegeben. Man rührt bei 45°C bis eine klare Lösung vorliegt. 20 mL Toluol werden abdestilliert, um Wasserspuren zu entfernen. Anschließend gibt man mit jeweils einer Spritze 0,738 g Diphenylmethandiisocyanat (MDI, 2,92 mmol) und eine Lösung von 120 µL 1,8-Diazabicyclo[5.4.0)undec-7-en (DBU) in 2 mL Toluol zu. Die Temperatur wird wieder auf 45°C erhöht. Nach 2 Std. entfernt man das Toluol am Rotationsverdampfer. Anschließend trocknet man bei 45°C (Wasserbad) am Feinvakuum bis zur Gewichtskonstanz.

### Beispiel 4: Herstellung von mit Hexamethylendiisocyanat kettenverlängertem Poloxamer aus drei verschiedenen Poloxameren

In einen Glaskolben mit Rückflusskühler werden 11,6 g Poloxamer 403 (Pluronic® P-123, 2 mmol), 12,5 g Poloxamer 407 (Pluronic® F-127; 1 mmol) und 14,6 g Poloxamer 338 (Pluronic® F-108, 1 mmol) eingewogen und auf 90 g mit trockenem Toluol aufgefüllt. Man rührt mit Magnetrührfisch bei ca. 45°C, bis eine klare Lösung vorliegt.

20 mL Toluol werden abdestilliert, um Wasserspuren zu entfernen. Anschließend gibt man jeweils mit einer Spritze 0,665 g Hexamethylendiisocyanat (HMDI, 3,95 mmol) und eine Lösung von 120 mL 1,8-Diazabicyclo[5.4.0]undec-7-en (DBU) in 5 mL Toluol zu. Die Temperatur wird auf 55°C erhöht. Nach 2 Std. entfernt man das Toluol am Rotationsverdampfer. Anschließend trocknet man bei 45°C (Wasserbad) am Feinvakuum bis zur Gewichtskonstanz. Es wird ein relativ festes Polymer erhalten, das sich bei Temperaturen unter 10°C gut in Wasser löst.

Bereits eine 10%ige Lösung des so erhaltenen Polymers erstarrt beim Erwärmen auf 35°C und ergibt ein transparentes Gel.

Das in Beispiel 4 erhaltene kettenverlängerte Poloxamer aus drei verschiedenen Poloxameren besitzt gegenüber einem kettenverlängerten Poloxamer, das ausschließlich aus Poloxamer 403 (Pluronic® P 123) hergestellt ist, den Vorteil, dass es transparent ist und bei geringen Konzentrationen erstarrt. Gegenüber einem kettenverlängerten Poloxamer, das ausschließlich aus Poloxamer 407 (Pluronic® F 127) hergestellt ist, ist hervorzuheben, dass sich das neue Material auch nach mehreren Tagen bei 37°C nicht in überstehendem Kulturmedium löst.

### Beispiel 5: Eigenschaften der thermoreversiblen Hydrogele aus kettenverlängerten Poloxameren

Die Materialien zeigen im Temperaturbereich zwischen 15° und 35°C zwei charakteristische Veränderungen. Beim Erwärmen von 4°C an erfolgt zwischen 15° und 20°C zunächst eine Trübung der bis dahin transparenten Flüssigkeiten (Sol). In Fig. 1a ist hier das Verhalten eines thermoreversiblen Hydrogels aus 10% eines mit Hexamethylendiisocyanat kettenverlängerten Poloxamers 403 wiedergegeben.

Dann steigt zwischen 22° und 35°C die Viskosität um mehrere Grössenordnungen an. Als Beispiele zeigt Fig. 1b jeweils 10%ige Lösungen von mit Hexamethylendiisocyanat kettenverlängertem Poloxamer 403 (gekennzeichnet als PMT001), mit Butandiisocyanat kettenverlängertem Poloxamer 403 (PMT002) und mit Methylenbiscyclohexyldiisocyanat kettenverlängertem Poloxamer 403 (PMT003). Letzteres hat weniger Wiederholungsein heiten.

Fig. 1c zeigt für dieselben thermoreversiblen Hydrogele PMT001 bis PMT003, dass der Eindringwiderstand einen ähnlichen Verlauf mit einem klaren Maximum zwischen 30° und 35°C besitzt.

Fig. 1d zeigt, dass mit steigender Konzentration erwartungsgemäß die Festigkeit der Gele (25%) zunimmt.

Fig. 1e zeigt, dass sich die Gele zur gleichmäßigen Proteinfreisetzung über mehrere Wochen eignen. Bei der gezeigten Messung wurde mit Hexamethylendiisocyanat kettenverlängertes Poloxamer 403 bei Konzentrationen von 20% bzw. 25% verwendet.

### Beispiel 6: Effekt der Gelkonzentration

Die gewünschte Menge des kettenverlängerten Poloxamers (Polymers) wird unter sterilen Bedingungen eingewogen und mit der entsprechenden Menge an Zellkulturmedium versetzt. Beides wird in einer Spritze bei 4°C über einige Tage gelagert und immer wieder gerührt. Dabei bildet sich eine homogene Lösung, die bei ca. 25 bis 30°C geliert. Bei ca. 15°C werden 100.000 immortalisierte Stammzellen pro Milliliter zugefügt. Die Zellzahl wird in einer Neubauer-Zählkammer bestimmt, die Zellen werden in ein Falcontube zu einem Pellet zentrifugiert und dann in die Mischung gegeben. Unterschiedliche Polymerkonzentrationen (2,5%, 5% und 10%) werden zunächst stündlich, dann täglich und schließlich noch nach einer Woche mittels "Live/Dead-Assay" untersucht. Es werden sowohl an der Unterseite als auch im Inneren der Probe mikroskopische Untersuchungen durchgeführt. Dabei ist das Zellüberleben bei 10% Polymerkonzentration etwas geringer als bei den niedrigeren Konzentrationen. Andererseits ist die Festigkeit des Gels deutlich höher.

Die Ergebnisse sind in Fig. 2 und 3 dargestellt.

### Beispiel 7: Effekt des biologischen Materials am Beispiel von Kollagen

Um die hohe biologische Aktivität des thermoreversiblen Gels mit biologischem Material zu demonstrieren wurde eine Reihe von Versuchen durchgeführt, bei denen 100.000 immortalisierte Stammzellen pro Milliliter mit verschiedenen Konzentrationen (0%, 10% und 20%) von löslichem Kollagen in einem 10%igen Hydrogel der Erfindung kultiviert wurden. Die Kultur erfolgt bei 37°C in feuchter Atmosphäre mit 5% Kohlendioxid und 95% Luft. Die Vitalität wurde über 7 Tage mittels "Live/Dead- Assay" ermittelt. Zudem wurde die Zellmorphologie mit einem konfokalen Laser- Scanning-Mikroskop untersucht. Die Ergebnisse sind in Fig. 4 und Fig. 5 dargestellt.
1. Eine 10%ige Lösung des Polymers aus mit 1,4-Butandiisocyanat kettenverlängertem Poloxamer 403 (vgl. Beispiel 2) in Zellkulturmedium wird wie oben beschrieben hergestellt. 100.000 eGFP-SCP1-Zellen werden in 1 mL dieser Lösung suspendiert. Die Zellzahl wurde in einer Neubauer Zählkammer bestimmt, die Zellen werden in ein Falcontube zu einem Pellet zentrifugiert und dann in das Gel überführt. Nach 1, 2, 3 und 7 Tagen werden die Proben untersucht. Das Zellenüberleben (vgl. Fig. 4 links) ist mit 45% nach 7 Tagen akzeptabel, doch zeigen die Zellen eine runde Morphologie, was nicht auf eine verstärkte Adhärenz der Zellen schließen lässt, da auf letzteres nur bei ausgebreiteter Zellmorphologie geschlossen werden kann.
2. Es wird wie oben verfahren, allerdings werden 10% lösliches Kollagen aus Rattenschwänzen ("Coll Type I-rat tail", Merck) zur Lösung hinzugefügt (vgl. Fig. 4 Mitte). Hier ist das Zellüberleben mit ca. 70% nach 7 Tagen beträchtlich. Schon nach 3 Tagen zeigt sich die typische gestreckte Morphologie der Zellen.
3. Es wird verfahren wie unter 2., nur dass diesmal 20% Kollagen in die Mischung eingebracht werden (vgl. Fig. 4 rechts). Das Zellüberleben ist mit 80% hervorragend. Die Zellen zeigen eine noch ausgeprägtere längliche Morphologie.

Fig. 4 zeigt die zugehörigen Mikroskopaufnahmen, die das Zellüberleben von eGFP-SCP1s nach 7 Tagen im thermoreversiblen Hydrogel der vorliegenden Erfindung mit bzw. ohne Kollagen I, veranschaulichen. Es werden signifikante Unterschiede zwischen thermoreversiblem Hydrogel der Erfindung ohne bzw. mit Kollagen I sichtbar. Beispielhafte Mikroskopbilder am Boden (Vergrößerung 10x) nach Anfärben mit Propidiumiodid; der Maßstab (Maßstrich) beträgt 200 µm.

Fig. 5 zeigt eGFP-SCP1s-Zellen nach 7 Tagen im thermoreversiblen Hydrogel (in der Abbildung als "BDI-hydrogel" bezeichnet) mit bzw. ohne 20 % Kollagen I, visualisiert mit konfokaler Laser-Scanning-Mikroskopie. Die Aufnahmen wurden mit einer Vergrösserung von 10x (linke Seite) bzw. 63x (rechte Seite) vorgenommen.

### Beispiel 8: Effekt von Sauerstoff auf das Zellüberleben im Applikationssystem

### a) Applikationssystem mit unterschiedlichen Konzentrationen an sauerstofffreisetzender Substanz (CaO₂) ohne Enzym

hTERT-immortalisierte humane mesenchymale Stammzellen wurden in Spritzen mit thermoreversiblem Hydrogel, das aus Poloxamer 403 und Butandiisocyanat erhalten wurde (d.h. "BDI-Hydrogel") bei Konzentrationen von 1 mg/ml, 0,5 mg/ml, 0,25 mg/ml sowie 0,1 mg/ml Calciumperoxid (CaO₂) kultiviert, und die Sauerstoffsättigung wurde in stündlichem Abstand bestimmt. Im Anschluss wurden mittels Vitalitätsassays die Zellüberlebensraten ermittelt Die Ergebnisse sind in Fig. 6a gezeigt.

### b) Applikationssystem mit sauerstofffreisetzender Substanz (CaO₂) und unterschiedlichen Konzentrationen an Enzym

Beispiel 8a) wurde wiederholt, jedoch wurden als Enzym 100 U/ml bzw. 200 U/ml, bzw. keine Katalase (Kontrolle) hinzugefügt. Die Ergebnisse sind in Fig. 6b gezeigt, woraus ersichtlich wird, dass sich Katalase-Konzentration von 100 U/ml bzw. 200 U/ml sich vorteilhaft auf das Zellüberleben auswirken.

### c) Applikationssystem unterschiedlicher Konzentrationen an sauerstofffreisetzender Substanz bei konstanter Enzymkonzentration

Analog Beispiel 8b) wurde die Sauerstoffsättigung (A) und Zellüberleben (B) nach 72 h Kultur in Spritzen in BDI-Hydrogel mit 0.5 mg/ml CaO₂ und 100 U/ml Katalase bzw. BDI-Hydrogel mit 0,25 mg/ml CaO₂ und 100 U/ml Katalase gemessen. Die Ergebnisse sind in Fig. 6c gezeigt, woraus ersichtlich wird, dass bei einer Konzentration von 0,25 mg/ml Ca02 und einer Katalase-Konzentration von 100 U/ml selbst nach 72 Stunden das Zellüberleben mit einem Wert von ca. 70% gegenüber der mit "BDI- Hydrogel" bezeichneten Kontrolle verbessert ist.

## Patentansprüche

1. Thermoreversibles Hydrogel, umfassend
(a) ein kettenverlängertes Poloxamer P*, hergestellt durch Umsetzung von mindestens einem Poloxamer P, wobei jedes Poloxamer zwei Poly(oxyethylen)blöcke PEG und einen Poly(oxypropylen)block PPG umfasst und das Molekulargewicht des Poly(oxypropylen)-blocks von P grösser ist als 2350 Da, mit einem Diisocyanat mit der Maßgabe, dass bei einem Molekulargewicht Mw des Poloxamers P von 11.000 oder mehr und einem Anteil an Polyethylenoxid in P von mehr als 60% mindestens zwei verschiedene Poloxamere umgesetzt werden,
sowie
(b) ein biologisches Material, ausgewählt aus der Gruppe, bestehend aus einem antibiotischen, antimikrobiellen oder antimykotischen Wirkstoff, Proteinen, Glukosaminoglykanen, Lysozym und einer natürlichen oder synthetischen Polyaminosäure.

2. Thermoreversibles Hydrogel gemäß Anspruch 1, wobei bei der Herstellung des kettenverlängerten Poloxamers P* zwei oder drei verschiedene Poloxamere umgesetzt werden.

3. Thermoreversibles Hydrogel gemäß Anspruch 1, wobei das kettenverlängerte Poloxamer Struktureinheiten der folgenden Formel besitzt: 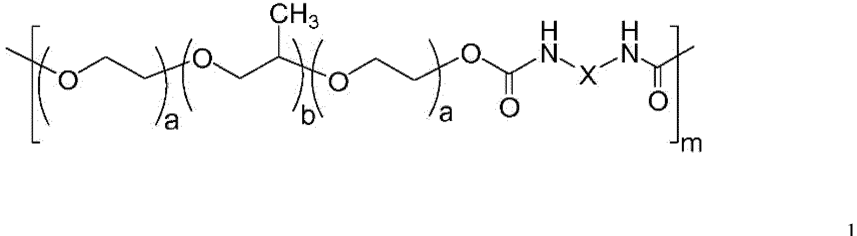
worin a und b jeweils ganze Zahlen zwischen 1 und 110 sind und m ≥ 3 ist; a bezeichnet die Anzahl Wiederholungseinheiten des Poly(oxyethylen)blocks PEG, und b bezeichnet die Anzahl Wiederholungseinheiten des Poly-(oxypropylen)blocks PPG; wobei das Diisocyanat die Formel O=C=N-X--N=C=O besitzt, und wobei X der aliphatische oder aromatische Rest des Diisocyanats ist;
und bei Verwendung von zwei verschiedenen Poloxameren P und P' das kettenverlängerte Poloxamer zusätzlich Struktureinheiten der Formel 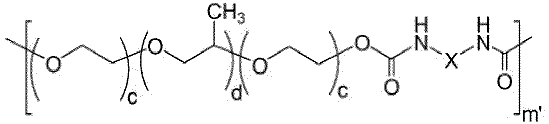
und/oder der Formel 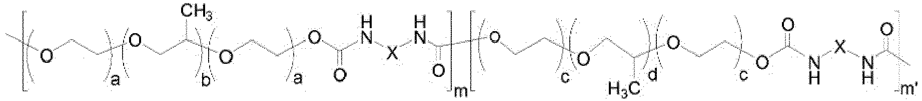 besitzt, wobei a, b, m sowie X wie oben definiert sind, und c die Anzahl Wiederholungseinheiten des Poly(ethylenoxid)blocks PEG, und d die Anzahl Wiederholungseinheiten des Poly(propylenoxid)blocks PPG jeweils im zweiten Poloxamer P' bezeichnet, wobei c und d jeweils ganze Zahlen zwischen 1 und 110 sind, und m' ≥ 3 ist.

4. Thermoreversibles Hydrogel gemäß einem der Ansprüche 1 bis 2, wobei bei der Herstellung des kettenverlängerten Poloxamers P* das Diisocyanat ausgewählt ist aus der Gruppe, bestehend aus aliphatischen Diisocyanaten und aromatischen Diisocyanaten.

5. Thermoreversibles Hydrogel gemäß Anspruch 4, wobei das Diisocyanat ausgewählt ist aus der Gruppe, bestehend aus linearen Diisocyanaten O=C=N-(CH₂)ₙ-N=C=O, wobei n = 4 bis 12 ist, Butandiisocyanat, Hexamethylendiisocyanat, Isophorondiisocyanat, Methylenbiscyclohexyldiisocyanat (H₁₂-MDI), Lysindiisocyanat (LDI), und Diphenylmethandiisocyanat (MDI).

6. Thermoreversibles Hydrogel gemäß Anspruch 1 bis 5, wobei das bzw. die Poloxamere aus den Poloxameren 272, 333, 334, 335, 402, 403 und 407 ausgewählt ist bzw. sind.

7. Thermoreversibles Hydrogel gemäß einem der Ansprüche 1 bis 6, wobei das kettenverlängerte Poloxamer P* gebildet wird durch Umsetzung des Poloxamers mit 0,8 bis 1,2 Äquivalenten Diisocyanat bei einer Temperatur von 40°C bis 60°C in Anwesenheit eines geeigneten Katalysators für die Bildung von Polyurethanen.

8. Thermoreversibles Hydrogel gemäß Anspruch 7, wobei P aus den Poloxameren definiert in Anspruch 6 ausgewählt ist.

9. Thermoreversibles Hydrogel gemäß einem der Ansprüche 1 bis 8, des Weiteren umfassend lebende Zellen.

10. Thermoreversibles Hydrogel gemäß Anspruch 9, wobei die lebenden Zellen ausgewählt sind aus der Gruppe, bestehend aus mononuklearen Zellen, mesenchymalen Stammzellen, deren Vorläuferzellen und daraus differenzierten Zellen, Osteoblasten, Adipozyten, Chondrozyten, Fibroblasten, Epithelzellen, Myoblasten, Tendozyten, mononuklearen hämatopoetischen Stamm- und Vorläuferzellen und daraus differenzierten Zellen, Immunzellen, multinuklearen Zellen; Riesenzellen, Makrophagen, und Osteoklasten.

11. Thermoreversibles Hydrogel gemäß Anspruch 9, wobei die Zellen genetisch modifiziert sind oder im Gel modifiziert werden.

12. Thermoreversibles Hydrogel gemäß einem der Ansprüche 1 bis 11, wobei das biologische Material eine synthetische Polyaminosäure oder ein antibiotischer, antimikrobieller oder antimykotischer Wirkstoff, und/oder ein das Zellwachstum und die Zelldifferenzierung hemmendes oder stimulierendes Protein ist;
und/oder wobei
das biologische Material ein Protein ist, ausgewählt aus der Gruppe, bestehend aus Fibrin, Gelatine, Kollagenen, und knochenmorphogenen Proteinen (BMP);
und/oder wobei
das Protein ein Wachstumsfaktor ist, ausgewählt aus der Gruppe,
bestehend aus Insulin-ähnlichem Wachstumsfaktor (IGF), Transforming growth factor (TGF), Platelet Derived Growth Factor (PDGF), Epidermalem Wachstumsfaktor (EGF), Fibroblaste-Wachstumsfaktor (FGF), Granulocyte-Macrophage Colony Stimulating Factor (GMCSF), Vascular Endothelial Growth Factor (VEGF), Hepatocyte Growth Factor (HGF), Interleukin-1B (IL-1B), Interleukin-8 (IL-8), Nervenwachstumsfaktor (NGF), und hämatopoetischen Wachstumsfaktoren wie Erythropoietin und koloniestimulierenden Faktoren wie G-CSF; und/oder wobei das Protein ein Hormon oder Enzym ist, bevorzugt Follistatin oder Myostatin;
und/oder
wobei das biologische Material Erythropoietin, G-CSF, Follistatin oder Myostatin ist;
und/oder
wobei das Protein ein Antikörper oder ein Immunoglobulin ist, ausgewählt aus der Gruppe, bestehend aus IgA, IgD, IgE, IgM, IgG, IgY, und IgW; oder
wobei das Glukosaminoglykan Hyaluronan ist.

13. Thermoreversibles Hydrogel gemäß Anspruch 11, wobei das biologische Material ausgewählt ist aus der Gruppe, bestehend aus -Lactam-Antibiotika, Tetracyclinen, Aminoglykosiden, Makroliden, Virustatika, Allylaminen, antimykotisch wirksamen Antibiotika, Imidazolen und -derivaten, Epidermalem Wachstumsfaktor (EGF), TGF- -(transforming growth factor beta)-Superfamilie, bevorzugt BMP (bone morhogenetic protein)- und GDF (growth differentiation factor)-Familien, stärker bevorzugt BMP2, BMP7 und GDF-5, löslichem Kollagen, fein gemahlenem Kollagen, Gelatine, menschlichem Blut, tierischem Blut, Komponenten menschlichen oder tierischen Blutes, und Kollagen Typ I, II oder III.

14. Thermoreversibles Hydrogel gemäß einem der Ansprüche 1 bis 13, wobei das kettenverlängerte Poloxamer hergestellt wird unter Verwendung eines linearen aliphatischen oder eines cyclischen, aliphatischen Diisocyanats.

15. Thermoreversibles Hydrogel gemäß einem der Ansprüche 1 bis 13, wobei das Hydrogel zusätzlich einen Tracer enthält, vorzugsweise einen ausgewählt aus radioaktiven Substanzen, die mittels PET oder SPECT sichtbar gemacht werden können.

16. Thermoreversibles Hydrogel gemäß einem der Ansprüche 1 bis 15 zur medizinischen Verwendung.

17. Thermoreversibles Hydrogel zur medizinischen Verwendung gemäß Anspruch 16, wobei die medizinische Verwendung Arzneimittelabgabe oder eine biomedizinische Anwendung ist.

18. Applikationssystem, umfassend das thermoreversible Hydrogel gemäß einem der Ansprüche 1 bis 16.

19. In-vitro-Verfahren zur Bildung einer Zusammensetzung auf einer Oberfläche, das die Applikation einer wässrigen Lösung eines thermoreversiblen Hydrogels gemäß Anspruch 1 bis 16, von Gemischen davon, oder des medizinischen Applikationssystems gemäß Anspruch 18 auf die Oberfläche umfasst, wobei das Verfahren gegebenenfalls die Applikation einer wässrigen Lösung eines lebende Zellen enthaltenden thermoreversiblen Hydrogels gemäß Anspruch 10 umfasst; und/oder
wobei das Applikationssystem gemäß Anspruch 18 verwendet wird und das Enzym Katalase ist, und/oder
wobei das thermoreversible Hydrogel hergestellt ist aus mit Butandiisocyanat kettenverlängertem Poloxamer 403, und/oder die sauerstofffreisetzende Substanz CaO₂ ist.

## Claims

1. Thermoreversible hydrogel including
(a) a chain-extended poloxamer P* prepared by reacting at least one poloxamer P with a diisocyanate, each poloxamer comprising two poly(oxyethylene) blocks PEG and one poly(oxypropylene) block PPG and the molecular weight of the poly(oxypropylene) block of P being greater than 2350 Da, with the proviso that at a molecular weight Mw of the poloxamer P of 11,000 or more and a proportion of polyethylene oxide in P exceeding 60%, at least two different poloxamers are reacted, and
(b) a biological material, selected from the group consisting of an antibiotic, antimicrobial or antimycotic active substance, proteins, glucosaminoglycans, lysozyme and a natural or synthetic polyamino acid.

2. Thermoreversible hydrogel according to claim 1, wherein for the preparation of the chain-extended poloxamer P* two or three different poloxamers are reacted.

3. Thermoreversible hydrogel according to claim 1, wherein the chain-extended poloxamer has structural units represented by the following formula: 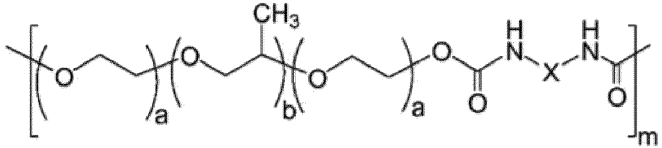
wherein a and b are each whole numbers between 1 and 110, and m ≥ 3; a denotes the number of repeat units of the poly(ethylene oxide) block PEG. and b denotes the number of repeat units of the poly(propyleneoxide) block PPG; the diisocyanate is represented by the formula O=C=N-X-N=C=O, and X represents the aliphatic or aromatic moiety of the diisocyanate;
and wherein, when using two different poloxamers P and P', the chain-extended poloxamer additionally has the structural units represented by the formula 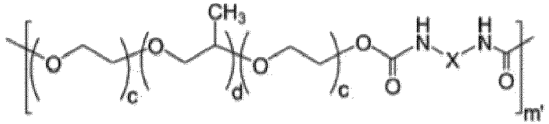
and/or the formula 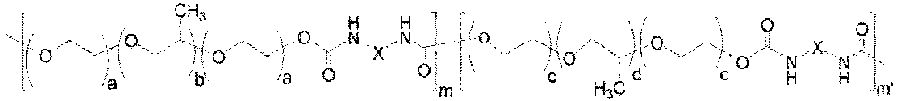 wherein a, b, m and X are as defined above, and c represents the number of repeat units of the poly(ethylene oxide) block PEG and d denotes the number of repeat units of the polypropylene oxide) block PPG, respectively referred to in the second poloxamer P', wherein c and d are each whole numbers between 1 and 110, and m' ≥ 3.

4. Thermoreversible hydrogel according to one of claims 1 to 2, wherein for the preparation of the chain-extended poloxamer P* the diisocyanate is selected from the group consisting of aliphatic diisocyanates and aromatic diisocyanates.

5. Thermoreversible hydrogel according to claim 4, wherein the diisocyanate is selected from the group consisting of linear diisocyanates O=C=N-(CH₂)ₙ-N=C=O, wherein n = 4 to 12, butane diisocyanate, hexamethylene diisocyanate, isophorone diisocyanate, methylene bis-cyclohexyl diisocyanate (H₁₂-MDI), lysine diisocyanate (LDI) and diphenylmethane diisocyanate (MDI).

6. Thermoreversible hydrogel according to claims 1 to 5, wherein the poloxamer(s) is or are elected from the poloxamers 272, 333, 334, 335, 402, 403 and 407.

7. Thermoreversible hydrogel according to any one of claims 1 to 6, wherein the chain-extended poloxamer P* is formed by the reaction of the poloxamer with from 0,8 to 1,2 equivalents of the diisocyanate at a temperature of 40°C to 60°C in the presence of a suitable catalyst for the formation of polyurethanes.

8. Thermoreversible hydrogel according to claim 7, wherein P is selected from the poloxamers according to claim 6.

9. Thermoreversible hydrogel according to any one of claims 1 to 8, further including living cells,

10. Thermoreversible hydrogel according to claim 9, wherein the living cells are selected from the group consisting of mononuclear cells, mesenchymal stem cells, progenitor cells thereof and cells differentiated therefrom, osteoblasts, adipocytes, chondrocytes, fibroblasts, epithelial cells, myoblasts, tendocytes, mononuclear haematopoietic stem and progenitor cells and cells differentiated therefrom, immune cells, multinucleated cells; giant cells, macrophages and osteoclasts.

11. Thermoreversible hydrogel according to claim 9, wherein the cells are genetically modified or modified in the gel.

12. Thermoreversible hydrogel according to any one of claims 1 to 11, wherein the biological material is a synthetic polyamino acid or an antibiotic, antimicrobial or antimycotic active substance, and/or a protein inhibiting or stimulating cell growth and cell differentiation;
and/or wherein
the biological material is a protein selected from the group consisting of fibrin, gelatine, collagens, and bone-morphogenetic proteins (BMP);
and/or wherein
the protein is a growth factor, selected from the group consisting of the insulin-like growth factor (IGF), the transforming growth factor (TGF), the platelet-derived growth factor (PDGF), the epidermal growth factor (EGF), the fibroblast growth factor (FGF), the granulocyte-macrophage colony-stimulating factor (GMCSF), the vascular endothelial growth factor (VEGF), the hepatocyte growth factor (HGF), interleukin-1B (IL- 1B), interleukin-8 (IL-8), the nerve growth factor (NGF) and haematopoietic growth factors such as erythropoietin and colony-stimulating factors such as G-CSF; and/or
wherein the protein is a hormone or enzyme, preferably follistatin or myostatin;
and/or
wherein the biological material is erythropoietin, G-CSF, follistatin or myostatin;
and/or
wherein the protein is an antibody or an immunoglobulin, selected from the group consisting of IgA, IgD, IgE, IgM, IgG, IgY and IgW; or
wherein the glucosaminoglycan is hyaluronan.

13. Thermoreversible hydrogel according to claim 11, where the biological material is selected from the group consisting of β-lactam antibiotics, tetracyclines, aminoglycosides, macrolides, antivirals, allylamines, antimycotic antibiotics, imidazoles and derivatives thereof, the epidermal growth factor (EGF), the TGF-β-(transforming growth factor beta)-superfamily, preferably the BMP (bone morphogenetic protein)- and the GDF (growth differentiation factor)-families, more preferably BMP2, BMP7 and GDF-5, soluble collagen, finely-ground collagen, gelatine, human blood, animal blood, components of human or animal blood and collagen type I, II or III.

14. Thermoreversible hydrogel according to any one of claims 1 to 13, wherein the chain-extended poloxamer is prepared by using a linear aliphatic or a cyclic, aliphatic diisocyanate.

15. Thermoreversible hydrogel according to any one of claims 1 to 13, wherein the hydrogel further contains a tracer, preferably one being selected from radioactive substances, which can be visualised by PET or SPECT.

16. Thermoreversible hydrogel according to any one of claims 1 to 15 for medical use.

17. Thermoreversible hydrogel for medical use according to claim 16, wherein the medical use is drug delivery or a biomedical application.

18. Application system comprising the thermoreversible hydrogel according to any one of claims 1 to 16.

19. In-vitro method for forming a composition on a surface, comprising the application of an aqueous solution of a thermoreversible hydrogel according to claims 1 to 16, of mixtures thereof, or of the medical application system according to claim 18 onto the surface, wherein the method optionally comprises applying an aqueous solution of a thermoreversible hydrogel containing living cells according to claim 10; and /or wherein the medical application system according to claim 18 is used and the enzyme is catalase; and/or
wherein the thermoreversible hydrogel is prepared from a poloxamer 403, which is chain-extended by butane diisocyanate, and/or the oxygen-releasing substance is CaO₂.

## Revendications

1. Hydrogel thermoréversible comprenant
(a) un poloxamère P* à chaîne allongée préparé en faisant réagir au moins un poloxamère P, dans lequel chaque poloxamère comprend deux blocs de poly(oxyéthylène) PEG et un bloc de poly(oxypropylène) PPG, et le poids moléculaire du bloc de poly(oxypropylène) de P est supérieur à 2350 Da, avec un diisocyanate, à condition qu'à un poids moléculaire Mw du poloxamère P de 11.000 ou plus et une proportion d'oxyde de polyéthylène dans le P supérieure à 60 %, au moins deux poloxamères différents sont mis à réagir, et
(b) un matériel biologique choisi parmi le groupe constitué par un antibiotique, un agent antimicrobien ou antifongique, des protéines, des glucosaminoglycanes, du lysozyme et un polyaminoacide naturel ou synthétique.

2. Hydrogel thermoréversible selon la revendication 1, dans lequel deux ou trois poloxamères différents sont mis à réagir lors de la préparation du poloxamère P* à chaîne allongée.

3. Hydrogel thermoréversible selon la revendication 1, dans lequel le poloxamère à chaîne allongée présente des unités structurelles de la formule suivante 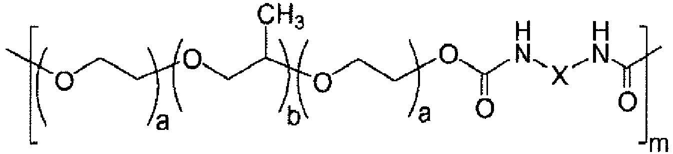
dans laquelle a et b sont chacun des nombres entiers compris entre 1 et 110 et m est ≥ 3 ; a désigne le nombre de motifs répétitifs de la séquence poly(oxyéthylène) PEG, et b désigne le nombre de motifs répétitifs de la séquence poly(oxypropylène) PPG ; dans laquelle le diisocyanate a la formule O=C=N-X--N=C=0, et dans laquelle X est le résidu aliphatique ou aromatique du diisocyanate ;
et lorsque deux poloxamères différents P et P' sont utilisés, le poloxamère à chaîne allongée contient en outre des unités structurelles de formule 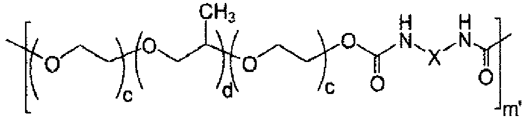
et/ou la formule 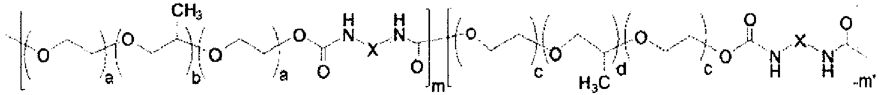 dans laquelle a, b, m et X sont tels que définis ci-dessus, et c est le nombre de motifs répétitifs du bloc poly(oxyde d'éthylène) PEG, et d est le nombre de motifs répétitifs du bloc poly(oxyde de propylène) PPG dans le second poloxamère P', dans lequel c et d sont chacun des nombres entiers compris entre 1 et 110, et m' est ≥ 3.

4. Hydrogel thermoréversible selon l'une quelconque des revendications 1 à 2, dans lequel, lors de la préparation du poloxamère P* à chaîne allongée, le diisocyanate est choisi parmi le groupe constitué par les diisocyanates aliphatiques et les diisocyanates aromatiques.

5. Hydrogel thermoréversible selon la revendication 4, dans lequel le diisocyanate est choisi parmi le groupe constitué par les diisocyanates linéaires O=C=N-(CH₂)ₙ-N=C=0, où n = 4 à 12, le diisocyanate de butane, le diisocyanate d'hexaméthylène, le diisocyanate d'isophorone, le diisocyanate de méthylènebiscyclohexyle (H₁₂-MDI), le diisocyanate de lysine (LD1) et le diisocyanate de diphénylméthane (MDI).

6. Hydrogel thermoréversible selon les revendications 1 à 5, dans lequel le ou les poloxamères, respectivement, sont choisi(s) parmi les poloxamères 272, 333, 334, 335, 402, 403 et 407.

7. Hydrogel thermoréversible selon l'une quelconque des revendications 1 à 6, dans lequel le poloxamère P* à chaîne allongée est formé par réaction du poloxamère avec 0,8 à 1,2 équivalents de diisocyanate à une température de 40°C à 60°C en présence d'un catalyseur approprié pour la formation de polyuréthanes.

8. Hydrogel thermoréversible selon la revendication 7, dans lequel P est choisi parmi les poloxamères définis dans la revendication 6.

9. Hydrogel thermoréversible selon l'une des revendications 1 à 8, comprenant en outre des cellules vivantes.

10. Hydrogel thermoréversible selon la revendication 9, dans lequel les cellules vivantes sont choisies parmi le groupe constitué par les cellules mononucléaires, les cellules souches mésenchymateuses, leurs cellules précurseurs et les cellules différenciées à partir de celles-ci, les ostéoblastes, les adipocytes, les chondrocytes, les fibroblastes, les cellules épithéliales, les myoblastes, les tendozymes, les cellules souches hématopoïétiques mononucléaires et les cellules précurseurs et les cellules différenciées à partir de celles-ci, les cellules immunitaires, les cellules multinucléaires ; les cellules géantes, les macrophages et les ostéoclastes.

11. Hydrogel thermoréversible selon la revendication 9, dans lequel les cellules sont génétiquement modifiées ou sont modifiées dans le gel.

12. Hydrogel thermoréversible selon l'une des revendications 1 à 11, dans lequel le matériau biologique est un polyaminoacide synthétique ou un antibiotique, un agent antimicrobien ou antifongique, et/ou une protéine qui inhibe ou stimule la croissance et la différenciation cellulaires ;
et/ou dans lequel
le matériel biologique est une protéine sélectionnée parmi le groupe composé de fibrine, de gélatine, de collagène et de protéines morphogènes osseuses (BMP) ;
et/ou dans lequel
la protéine est un facteur de croissance choisi parmi le groupe constitué par le facteur de croissance analogue à l'insuline (IGF), le facteur de croissance transformant (TGF), le facteur de croissance dérivé des plaquettes (PDGF), le facteur de croissance épidermique (EGF), le facteur de croissance des fibroblastes (FGF), le facteur de stimulation des colonies de granulocytes et de macrophages (GMCSF), le facteur de croissance de l'endothélium vasculaire (VEGF), le facteur de croissance des hépatocytes (HGF), l'interleukine-1 B (IL-1B), l'interleukine-8 (IL-8), le facteur de croissance des nerfs (NGF), et
les facteurs de croissance hématopoïétiques tels que l'érythropoïétine et les facteurs de stimulation des colonies tels que le G-CSF ; et/ou
dans lequel la protéine est une hormone ou une enzyme, de préférence la follistatine ou la myostatine ;
et/ou
dans lequel le matériel biologique est l'érythropoïétine, le G-CSF, la follistatine ou la myostatine ;
et/ou
dans lequel la protéine est un anticorps ou une immunoglobuline choisie parmi le groupe constitué par les IgA, IgD, IgE, IgM, IgG, IgY et IgW ; ou
dans lequel le glucosaminoglycane est l'hyaluronane.

13. Hydrogel thermoréversible selon la revendication 11, dans lequel le matériau biologique est choisi parmi le groupe constitué par les antibiotiques lactamines, les tétracyclines, les aminoglycosides, les macrolides, les agents antiviraux, les allylamines, les antibiotiques antifongiques, les imidazoles et leurs dérivés, le facteur de croissance épidermique (EGF), la superfamille des TGF (facteur de croissance transformant beta), de préférence les familles BMP (protéine morhogénétique osseuse) et GDF (facteur de différenciation de la croissance), plus préférablement BMP2, BMP7 et GDF5, le collagène soluble, le collagène finement broyé, la gélatine, le sang humain, le sang animal, les composants sanguins humains ou animaux et le collagène de type I, II ou III.

14. Hydrogel thermoréversible selon l'une quelconque des revendications 1 à 13, dans lequel le poloxamère à chaîne allongée est préparé en utilisant un diisocyanate aliphatique linéaire ou un diisocyanate aliphatique cyclique.

15. Hydrogel thermoréversible selon l'une quelconque des revendications 1 à 13, dans lequel l'hydrogel contient en outre un traceur, de préférence un traceur choisi parmi les substances radioactives qui peuvent être rendues visibles au moyen de la TEP ou de la TEMP.

16. Hydrogel thermoréversible selon l'une des revendications 1 à 15 pour usage médical.

17. Hydrogel thermoréversible à usage médical selon la revendication 16, dans lequel l'usage médical est l'administration de médicaments ou une application biomédicale.

18. Système d'application comprenant l'hydrogel thermoréversible selon l'une des revendications 1 à 16.

19. Méthode in vitro pour former une composition sur une surface, qui comprend l'application à la surface d'une solution aqueuse d'un hydrogel thermoréversible selon les revendications 1 à 16, de mélanges de ceux-ci, ou du système d'application médicale selon la revendication 18, dans laquelle la méthode comprend éventuellement l'application à la surface d'une solution aqueuse d'un hydrogel thermoréversible contenant des cellules vivantes selon la revendication 10 ; et/ou dans laquelle le système d'application selon la revendication 18 est utilisé et l'enzyme est la catalase, et/ou
dans lequel l'hydrogel thermoréversible est préparé à partir de poloxamère 403 à chaîne allongée de diisocyanate de butane et/ou de la substance libérant de l'oxygène CaO2.
